(19) Europäisches Patentamt
European Patent Office
Office européen des brevets

(11) EP 3 882 276 A1

(12) EUROPEAN PATENT APPLICATION
published in accordance with Art. 153(4) EPC

(43) Date of publication:
**22.09.2021 Bulletin 2021/38**

(21) Application number: **20773644.8**

(22) Date of filing: **25.02.2020**

(51) Int Cl.:
*C07K 16/46* (2006.01) *C12N 15/13* (2006.01)
*A61K 39/395* (2006.01) *A61P 35/00* (2006.01)
*A61P 37/02* (2006.01) *G01N 33/68* (2006.01)

(86) International application number:
**PCT/CN2020/076516**

(87) International publication number:
**WO 2020/186974 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2019 CN 201910209330**

(71) Applicant: **Excyte LLC**
**Beijing 100085 (CN)**

(72) Inventors:
- **YUAN, Andy Qingan**
  **Beijing 100085 (CN)**
- **MENG, Qingwu**
  **Beijing 100085 (CN)**
- **BAI, Lili**
  **Beijing 100085 (CN)**
- **ZHAO, Likun**
  **Beijing 100085 (CN)**
- **LI, Yanhu**
  **Beijing 100085 (CN)**

(74) Representative: **Raffay & Fleck Patentanwälte**
**Grosse Bleichen 8**
**20354 Hamburg (DE)**

# (54) BISPECIFIC ANTIBODY, PREPARATION METHOD THEREFOR AND APPLICATION THEREOF

(57) The present invention relates to a bispecific antibody, a preparation method therefor and an application thereof. The bispecific antibody in the present invention comprises a monoclonal antibody unit and a single-chain antibody unit. The single-chain antibody unit comprises two complete light chain-heavy chain pairs, and is specifically bound to a surface antigen of a tumor cell. The single-chain antibody unit comprises two single-chain antibodies. The single-chain antibody comprises a heavy chain variable region and a light chain variable region, and is specifically bound to a surface antigen of an immunocyte. The bispecific antibody provided in the present invention is of a symmetric structure formed by binding by means of any one of the following modes: (1) C-ends of the two single-chain antibodies are respectively bound to N-ends of two heavy chains of a monoclonal antibody by means of a connecting peptide; (2) N-ends of the two single-chain antibodies are respectively bound to C-ends of the two heavy chains of the monoclonal antibody by means of the connecting peptide. The bispecific antibody in the present invention can be simultaneously bound to the immunocyte and the tumor cell, can mediate a directed immune response, and can effectively kill the tumor cell.

Figure 2

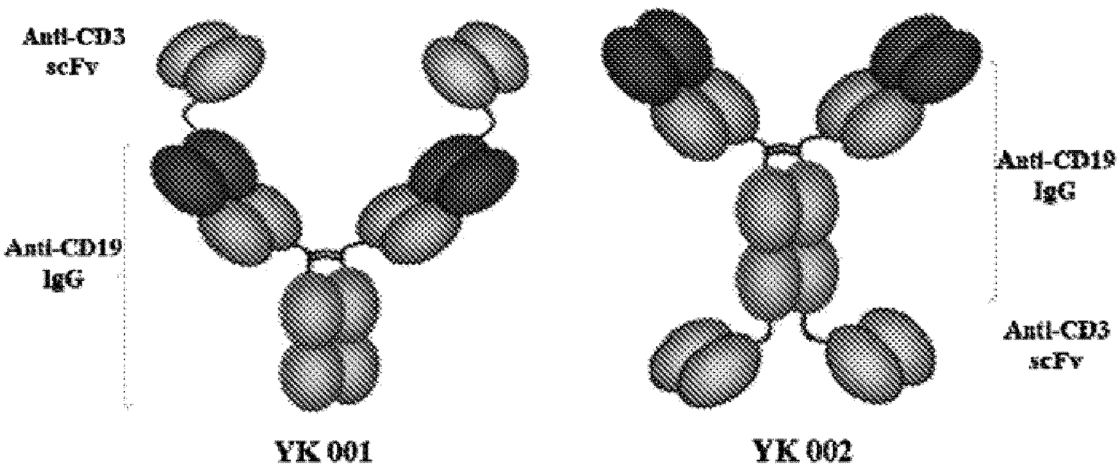
Anti-CD3
scFv
Anti-CD19
IgG
YK 001
Anti-CD19
IgG
Anti-CD3
scFv
YK 002

A B

Sequence Listing

<110> EXCYTE LLC

<120> BISPECIFIC ANTIBODY, PREPARATION METHOD THEREOF AND APPLICATION THEREOF

<130> KHP209810004.4

<160> 21

<170> SIPO Sequence Listing 1.0

<210> 1
<211> 451
<212> PRT
<213> Artificial Sequence

<220>
<223> Laboratory synthesis

<400> 1
Gln Val Gln Leu Gln Gln Ser Gly Ala Glu Leu Val Arg Pro Gly Ser
1 5 10
15
Ser Val Lys Ile Ser Cys Lys Ala Ser Gly Tyr Ala Phe Ser Ser Tyr
20 25 30

Trp Met Asn Trp Val Lys Gln Arg Pro Gly Gln Gly Leu Glu Trp Ile
35 40 45

Gly Gln Ile Trp Pro Gly Asp Gly Asp Thr Asn Tyr Asn Gly Lys Phe
50 55 60

Lys Gly Lys Ala Thr Leu Thr Ala Asp Glu Ser Ser Ser Thr Ala Tyr
65 70 75
80
Met Gln Leu Ser Ser Leu Ala Ser Glu Asp Ser Ala Val Tyr Phe Cys
85 90
95
Ala Arg Arg Glu Thr Thr Thr Val Gly Arg Tyr Tyr Tyr Ala Met Asp
100 105 110

Tyr Trp Gly Gln Gly Thr Thr Val Thr Val Ser Ser Ala Ser Thr Lys
115 120 125

Gly Pro Ser Val Phe Pro Leu Ala Pro Cys Ser Arg Ser Thr Ser Glu
130 135 140

Ser Thr Ala Ala Leu Gly Cys Leu Val Lys Asp Tyr Phe Pro Glu Pro
145 150 155
160
Val Thr Val Ser Trp Asn Ser Gly Ala Leu Thr Ser Gly Val His Thr
165 170
175
Phe Pro Ala Val Leu Gln Ser Ser Gly Leu Tyr Ser Leu Ser Ser Val
180 185
190
Val Thr Val Pro Ser Ser Ser Leu Gly Thr Lys Thr Tyr Thr Cys Asn
195 200 205

Val Asp His Lys Pro Ser Asn Thr Lys Val Asp Lys Arg Val Glu Ser
210 215 220

```
Lys Tyr Gly Pro Pro Cys Pro Ser Cys Pro Ala Pro Glu Phe Leu Gly
225                 230                 235
240
Gly Pro Ser Val Phe Leu Phe Pro Pro Lys Pro Lys Asp Thr Leu Met
                245                 250
255
Ile Ser Arg Thr Pro Glu Val Thr Cys Val Val Val Asp Val Ser Gln
            260                 265                 270

Glu Asp Pro Glu Val Gln Phe Asn Trp Tyr Val Asp Gly Val Glu Val
        275                 280                 285

His Asn Ala Lys Thr Lys Pro Arg Glu Glu Gln Phe Asn Ser Thr Tyr
    290                 295                 300

Arg Val Val Ser Val Leu Thr Val Leu His Gln Asp Trp Leu Asn Gly
305                 310                 315
320
Lys Glu Tyr Lys Cys Lys Val Ser Asn Lys Gly Leu Pro Ser Ser Ile
                325                 330
335
Glu Lys Thr Ile Ser Lys Ala Lys Gly Gln Pro Arg Glu Pro Gln Val
            340                 345                 350

Tyr Thr Leu Pro Pro Ser Gln Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395
400
Val Leu Asp Ser Asp Gly Ser Phe Phe Leu Tyr Ser Arg Leu Thr Val
                405                 410
415
Asp Lys Ser Arg Trp Gln Glu Gly Asn Val Phe Ser Cys Ser Val Met
            420                 425
430
His Glu Ala Leu His Asn His Tyr Thr Gln Lys Ser Leu Ser Leu Ser
        435                 440                 445

Pro Gly Lys
    450

<210>  2
<211>  1353
<212>  DNA
<213>  Artificial Sequence

<220>
<223> Laboratory synthesis

<400>  2
caagttcaac ttcagcagtc tggcgccgaa ctcgtgcggc ctggatctag cgtgaagatc      60
agctgtaaag ccagcggcta cgccttcagc agctactgga tgaactgggt caagcagagg     120
cctggacagg gcctcgaatg gatcggacaa atttggcctg gcgacggcga caccaactac     180
aacggcaagt tcaagggcaa agccacactg accgccgacg agtctagcag cacagcctac     240
atgcagctga gcagcctggc ctctgaagat agcgccgtgt acttctgcgc cagacgggaa     300
acaaccaccg tgggcagata ttactacgcc atggactact ggggccaggg caccacagtg     360
acagttagct ctgcgtcgac caagggcccc agcgtgttcc ccctggcccc ttgcagcaga     420
agcaccagcg agagcacagc cgccctgggc tgcctggtga aggactactt ccccgagccc     480
```

gtgaccgtgt cctggaacag cggcgctctg accagcggcg tgcatacctt ccccgccgtg 540
ctccagagca gcggactgta ctccctgagc agcgtggtga ccgtgccttc cagcagcctg 600
ggcaccaaga cctacacttg caacgtggac cacaagccca gcaacaccaa ggtggacaag 660
agagtggaga gcaagtacgg ccccccatgc ccatcatgcc cagcacctga gttcctgggg 720
ggaccatcag tcttcctgtt ccccccaaaa cccaaggaca ctctcatgat ctcccggacc 780
cctgaggtca cgtgcgtggt ggtggacgtg agccaggaag accccgaggt ccagttcaac 840
tggtacgtgg atggcgtgga ggtgcataat gccaagacaa agccgcggga ggagcagttc 900
aacagcacgt accgtgtggt cagcgtcctc accgtcctgc accaggactg gctgaacggc 960
aaggagtaca agtgcaaggt ctccaacaaa ggcctcccgt cctccatcga gaaaaccatc 1020
tccaaagcca aagggcagcc ccgagagcca caggtgtaca ccctgccccc atcccaggag 1080
gagatgacca agaaccaggt cagcctgacc tgcctggtca aaggcttcta ccccagcgac 1140
atcgccgtgg agtgggagag caatgggcag ccggagaaca actacaagac cacgcctccc 1200
gtgctggact ccgacggctc cttcttcctc tacagcaggc taaccgtgga caagagcagg 1260
tggcaggagg ggaatgtctt ctcatgctcc gtgatgcatg aggctctgca caaccactac 1320
acacagaaga gcctctccct gtctccgggt aaa 1353

<210> 3
<211> 218
<212> PRT
<213> Artificial Sequence

<220>
<223> Laboratory synthesis

<400> 3
Asp Ile Gln Leu Thr Gln Ser Pro Ala Ser Leu Ala Val Ser Leu Gly
1 5 10
15
Gln Arg Ala Thr Ile Ser Cys Lys Ala Ser Gln Ser Val Asp Tyr Asp
20 25 30

Gly Asp Ser Tyr Leu Asn Trp Tyr Gln Gln Ile Pro Gly Gln Pro Pro
35 40 45

Lys Leu Leu Ile Tyr Asp Ala Ser Asn Leu Val Ser Gly Ile Pro Pro
50 55 60

Arg Phe Ser Gly Ser Gly Ser Gly Thr Asp Phe Thr Leu Asn Ile His
65 70 75
80
Pro Val Glu Lys Val Asp Ala Ala Thr Tyr His Cys Gln Gln Ser Thr
85 90
95
Glu Asp Pro Trp Thr Phe Gly Gly Gly Thr Lys Leu Glu Ile Lys Arg
100 105 110

Thr Val Ala Ala Pro Ser Val Phe Ile Phe Pro Pro Ser Asp Glu Gln
115 120 125

Leu Lys Ser Gly Thr Ala Ser Val Val Cys Leu Leu Asn Asn Phe Tyr
130 135 140

Pro Arg Glu Ala Lys Val Gln Trp Lys Val Asp Asn Ala Leu Gln Ser
145 150 155
160
Gly Asn Ser Gln Glu Ser Val Thr Glu Gln Asp Ser Lys Asp Ser Thr
165 170
175
Tyr Ser Leu Ser Ser Thr Leu Thr Leu Ser Lys Ala Asp Tyr Glu Lys
180 185 190

His Lys Val Tyr Ala Cys Glu Val Thr His Gln Gly Leu Ser Ser Pro
195 200 205

Val Thr Lys Ser Phe Asn Arg Gly Glu Cys
210 215

<210> 4
<211> 657
<212> DNA
<213> Artificial Sequence

<220>
<223> Laboratory synthesis

<400> 4
gatatccagc tgacacagag ccctgccagc ctggccgttt ctctgggaca gagagccacc 60
atcagctgca aggccagcca gagcgttgac tacgacggcg acagctacct gaactggtat 120
cagcagatcc ccggccagcc tcctaagctg ctgatctacg atgccagcaa cctggtgtct 180
ggcatccctc ctagatttc cggcagcggc tctggcaccg acttcaccct gaatatccat 240
cctgtggaaa aggtggacgc cgccacctac cactgtcagc agtctacaga ggacccctgg 300
acatttggcg gaggcaccaa gctggaaatc aagcgtacgg tggctgcacc atctgtcttc 360
atcttcccgc catctgatga gcagttgaaa tctggaactg cctctgttgt gtgcctgctg 420
aataacttct atcccagaga ggccaaagta cagtggaagg tggataacgc cctccaatcg 480
ggtaactccc aggagagtgt cacagagcag gacagcaagg acagcaccta cagcctcagc 540
agcaccctga cgctgagcaa agcagactac gagaaacaca aagtctacgc ctgcgaagtc 600
acccatcagg gcctgagctc gcccgtcaca aagagcttca acaggggaga gtgttag 657

<210> 5
<211> 106
<212> PRT
<213> Artificial Sequence

<220>
<223> Laboratory synthesis

<400> 5
Gln Ile Val Leu Thr Gln Ser Pro Ala Ile Met Ser Ala Ser Pro Gly
1 5 10
15
Glu Lys Val Thr Met Thr Cys Ser Ala Ser Ser Ser Val Ser Tyr Met
20 25 30

Asn Trp Tyr Gln Gln Lys Ser Gly Thr Ser Pro Lys Arg Trp Ile Tyr
35 40 45

Asp Thr Ser Lys Leu Ala Ser Gly Val Pro Ala His Phe Arg Gly Ser
50 55 60

Gly Ser Gly Thr Ser Tyr Ser Leu Thr Ile Ser Gly Met Glu Ala Glu
65 70 75
80
Asp Ala Ala Thr Tyr Tyr Cys Gln Gln Trp Ser Ser Asn Pro Phe Thr
85 90
95
Phe Gly Cys Gly Thr Lys Leu Glu Ile Lys
100 105

<210> 6
<211> 119
<212> PRT
<213> Artificial Sequence

<220>
<223> Laboratory synthesis

<400> 6
Gln Val Gln Leu Gln Gln Ser Gly Ala Glu Leu Ala Arg Pro Gly Ala
1 5 10
15
Ser Val Lys Met Ser Cys Lys Ala Ser Gly Tyr Thr Phe Thr Arg Tyr
20 25
30
Thr Met His Trp Val Lys Gln Arg Pro Gly Gln Cys Leu Glu Trp Ile
35 40 45

Gly Tyr Ile Asn Pro Ser Arg Gly Tyr Thr Asn Tyr Asn Gln Lys Phe
50 55 60

Lys Asp Lys Ala Thr Leu Thr Thr Asp Lys Ser Ser Ser Thr Ala Tyr
65 70 75
80
Met Gln Leu Ser Ser Leu Thr Ser Glu Asp Ser Ala Val Tyr Tyr Cys
85 90
95
Ala Arg Tyr Tyr Asp Asp His Tyr Cys Leu Asp Tyr Trp Gly Gln Gly
100 105 110

Thr Thr Leu Thr Val Ser Ser
115

<210> 7
<211> 318
<212> DNA
<213> Artificial Sequence

<220>
<223> Laboratory synthesis

<400> 7
cagatcgtgc tgacacagag ccccgccatc atgagtgcta gccctggcga gaaagtgacc 60
atgacctgta gcgccagcag cagcgtgtcc tacatgaact ggtatcagca gaagtccggc 120
acaagcccca agcggtggat ctacgataca agcaagctgg cctctggcgt gcccgctcac 180
tttagaggat ctggcagcgg caccagctac agcctgacaa tctctggcat ggaagccgag 240
gatgccgcca cctactattg ccagcagtgg tccagcaatc ccttcacctt tggctgtggc 300
accaagctgg aaatcaaa 318

<210> 8
<211> 357
<212> DNA
<213> Artificial Sequence

<220>
<223> Laboratory synthesis

<400> 8
caggttcagc tgcaacagtc tggcgccgaa cttgctagac caggcgccag cgtgaagatg 60
agctgtaaag ccagcggcta caccttcacc agatacacca tgcactgggt caagcagagg 120
cccggacagt gccttgagtg gatcggctac atcaacccca gccggggcta caccaactac 180
aaccagaagt tcaaggacaa ggccacactg accaccgaca agagcagcag cacagcctac 240
atgcagctga gcagcctgac cagcgaagat agcgccgtgt actactgcgc ccggtactac 300
gacgatcact actgcctgga ttactggggc cagggcacaa ccctgacagt gtcatct 357

<210> 9
<211> 107
<212> PRT
<213> Artificial Sequence

<220>

<223> Laboratory synthesis

<400> 9
Asp Ile Gln Met Thr Gln Ser Pro Ser Ser Leu Ser Ala Ser Val Gly
1 5 10
15
Asp Arg Val Thr Ile Thr Cys Arg Ala Ser Gln Asp Ile Arg Asn Tyr
20 25 30

Leu Asn Trp Tyr Gln Gln Lys Pro Gly Lys Ala Pro Lys Leu Leu Ile
35 40 45

Tyr Tyr Thr Ser Arg Leu Glu Ser Gly Val Pro Ser Arg Phe Ser Gly
50 55 60

Ser Gly Ser Gly Thr Asp Tyr Thr Leu Thr Ile Ser Ser Leu Gln Pro
65 70 75
80
Glu Asp Phe Ala Thr Tyr Tyr Cys Gln Gln Gly Asn Thr Leu Pro Trp
85 90
95
Thr Phe Gly Cys Gly Thr Lys Val Glu Ile Lys
100 105

<210> 10
<211> 122
<212> PRT
<213> Artificial Sequence

<220>
<223> Laboratory synthesis

<400> 10
Glu Val Gln Leu Val Glu Ser Gly Gly Gly Leu Val Gln Pro Gly Gly
1 5 10
15
Ser Leu Arg Leu Ser Cys Ala Ala Ser Gly Tyr Ser Phe Thr Gly Tyr
20 25
30
Thr Met Asn Trp Val Arg Gln Ala Pro Gly Lys Cys Leu Glu Trp Val
35 40 45

Ala Leu Ile Asn Pro Tyr Lys Gly Val Thr Thr Tyr Ala Asp Ser Val
50 55 60

Lys Gly Arg Phe Thr Ile Ser Val Asp Lys Ser Lys Asn Thr Ala Tyr
65 70 75
80
Leu Gln Met Asn Ser Leu Arg Ala Glu Asp Thr Ala Val Tyr Tyr Cys
85 90
95
Ala Arg Ser Gly Tyr Tyr Gly Asp Ser Asp Trp Tyr Phe Asp Val Trp
100 105 110

Gly Gln Gly Thr Leu Val Thr Val Ser Ser
115 120

<210> 11
<211> 321
<212> DNA
<213> Artificial Sequence

<220>
<223> Laboratory synthesis

<400> 11
gatatccaga tgacacagag ccctagcagc ctgtctgcca gcgtgggaga cagagtgacc 60
atcacctgta gagccagcca ggacatccgg aactacctga actggtatca gcagaagccc 120
ggcaaggccc ctaagctgct gatctactac accagcagac tggaaagcgg cgtgcccagc 180
agattttctg gcagcggcag cggaaccgac tacaccctga ccatatctag cctgcagcct 240
gaggacttcg ccacctacta ttgccagcag ggcaacaccc tgccttggac ctttggctgt 300
ggcaccaagg tggaaatcaa a 321

<210> 12
<211> 366
<212> DNA
<213> Artificial Sequence

<220>
<223> Laboratory synthesis

<400> 12
gaagtgcagc tggttgaatc aggcggaggc ctggttcagc caggcggatc tctgagactg 60
tcttgtgccg cctccggcta cagctttacc ggctacacca tgaattgggt ccgacaggcc 120
cctggcaagt gcctggaatg ggttgccctg atcaacccct acaagggcgt gaccacatac 180
gccgactctg tgaagggcag attcaccatc agcgtggaca agagcaagaa caccgcctac 240
ctgcagatga acagcctgag agccgaggac accgccgtgt attattgcgc cagaagcggc 300
tactacggcg acagcgactg gtactttgat gtgtggggac agggaaccct ggtcaccgtg 360
tctagc 363

<210> 13
<211> 15
<212> PRT
<213> Artificial Sequence

<220>
<223> Laboratory synthesis

<400> 13
Gly Gly Gly Gly Ser Gly Gly Gly Gly Ser Gly Gly Gly Gly Ser
1 5 10
15

<210> 14
<211> 735
<212> DNA
<213> Artificial Sequence

<220>
<223> Laboratory synthesis

<400> 14
cagatcgtgc tgacacagag ccccgccatc atgagtgcta gccctggcga gaaagtgacc 60
atgacctgta gcgccagcag cagcgtgtcc tacatgaact ggtatcagca gaagtccggc 120
acaagcccca agcggtggat ctacgataca agcaagctgg cctctggcgt gcccgctcac 180
tttagaggat ctggcagcgg caccagctac agcctgacaa tctctggcat ggaagccgag 240
gatgccgcca cctactattg ccagcagtgg tccagcaatc ccttcacctt tggctgtggc 300
accaagctgg aaatcaaagg cggaggcgga agtggcggcg gaggtagcgg tggtggtgga 360
agcggtggcg gaggatctca ggttcagctg caacagtctg gcgccgaact tgctagacca 420
ggcgccagcg tgaagatgag ctgtaaagcc agcggctaca ccttcaccag atacaccatg 480
cactgggtca agcagaggcc cggacagtgc cttgagtgga tcggctacat caaccccagc 540
cggggctaca ccaactacaa ccagaagttc aaggacaagg ccacactgac caccgacaag 600
agcagcagca cagcctacat gcagctgagc agcctgacca gcgaagatag cgccgtgtac 660
tactgcgccc ggtactacga cgatcactac tgcctggatt actggggcca gggcacaacc 720
ctgacagtgt catct 735

<210> 15
<211> 732
<212> DNA
<213> Artificial Sequence

<220>
<223> Laboratory synthesis

<400> 15
gatatccaga tgacacagag ccctagcagc ctgtctgcca gcgtgggaga cagagtgacc 60
atcacctgta gagccagcca ggacatccgg aactacctga actggtatca gcagaagccc 120
ggcaaggccc ctaagctgct gatctactac accagcagac tggaaagcgg cgtgcccagc 180
agatttctg gcagcggcag cggaaccgac tacaccctga ccatatctag cctgcagcct 240
gaggacttcg ccacctacta ttgccagcag ggcaacaccc tgccttggac ctttggctgt 300
ggcaccaagg tggaaatcaa aggtggtggc ggatctggtg gtggtggaag tggcggtggc 360
ggttctgaag tgcagctggt tgaatcaggc ggaggcctgg ttcagccagg cggatctctg 420
agactgtctt gtgccgcctc cggctacagc tttaccggct acaccatgaa ttgggtccga 480
caggcccctg gcaagtgcct ggaatgggtt gccctgatca acccctacaa gggcgtgacc 540
acatacgccg actctgtgaa gggcagattc accatcagcg tggacaagag caagaacacc 600
gcctacctgc agatgaacag cctgagagcc gaggacaccg ccgtgtatta ttgcgccaga 660
agcggctact acggcgacag cgactggtac tttgatgtgt ggggacaggg aaccctggtc 720
accgtgtcta gc 732

<210> 16
<211> 245
<212> PRT
<213> Artificial Sequence

<220>
<223> Laboratory synthesis

<400> 16
Gln Ile Val Leu Thr Gln Ser Pro Ala Ile Met Ser Ala Ser Pro Gly
1 5 10
15
Glu Lys Val Thr Met Thr Cys Ser Ala Ser Ser Ser Val Ser Tyr Met
20 25
30
Asn Trp Tyr Gln Gln Lys Ser Gly Thr Ser Pro Lys Arg Trp Ile Tyr
35 40 45

Asp Thr Ser Lys Leu Ala Ser Gly Val Pro Ala His Phe Arg Gly Ser
50 55 60

Gly Ser Gly Thr Ser Tyr Ser Leu Thr Ile Ser Gly Met Glu Ala Glu
65 70 75
80
Asp Ala Ala Thr Tyr Tyr Cys Gln Gln Trp Ser Ser Asn Pro Phe Thr
85 90
95
Phe Gly Cys Gly Thr Lys Leu Glu Ile Lys Gly Gly Gly Gly Ser Gly
100 105 110

Gly Gly Gly Ser Gly Gly Gly Gly Ser Gly Gly Gly Gly Ser Gln Val
115 120 125

Gln Leu Gln Gln Ser Gly Ala Glu Leu Ala Arg Pro Gly Ala Ser Val
130 135 140

Lys Met Ser Cys Lys Ala Ser Gly Tyr Thr Phe Thr Arg Tyr Thr Met
145 150 155
160

```
His Trp Val Lys Gln Arg Pro Gly Gln Cys Leu Glu Trp Ile Gly Tyr
                        165                           170
175
Ile Asn Pro Ser Arg Gly Tyr Thr Asn Tyr Asn Gln Lys Phe Lys Asp
                 180                           185                 190

Lys Ala Thr Leu Thr Thr Asp Lys Ser Ser Ser Thr Ala Tyr Met Gln
            195                           200                       205

Leu Ser Ser Leu Thr Ser Glu Asp Ser Ala Val Tyr Tyr Cys Ala Arg
       210                           215                           220

Tyr Tyr Asp Asp His Tyr Cys Leu Asp Tyr Trp Gly Gln Gly Thr Thr
225                           230                           235
240
Leu Thr Val Ser Ser
                        245

<210>  17
<211>  244
<212>  PRT
<213>  Artificial Sequence

<220>
<223> Laboratory synthesis

<400>  17
Asp Ile Gln Met Thr Gln Ser Pro Ser Ser Leu Ser Ala Ser Val Gly
1                       5                             10
15
Asp Arg Val Thr Ile Thr Cys Arg Ala Ser Gln Asp Ile Arg Asn Tyr
                   20                           25                  30

Leu Asn Trp Tyr Gln Gln Lys Pro Gly Lys Ala Pro Lys Leu Leu Ile
              35                            40                          45

Tyr Tyr Thr Ser Arg Leu Glu Ser Gly Val Pro Ser Arg Phe Ser Gly
       50                            55                            60

Ser Gly Ser Gly Thr Asp Tyr Thr Leu Thr Ile Ser Ser Leu Gln Pro
65                            70                            75
80
Glu Asp Phe Ala Thr Tyr Tyr Cys Gln Gln Gly Asn Thr Leu Pro Trp
                          85                            90
95
Thr Phe Gly Cys Gly Thr Lys Val Glu Ile Lys Gly Gly Gly Gly Ser
                  100                           105                 110

Gly Gly Gly Gly Ser Gly Gly Gly Gly Ser Glu Val Gln Leu Val Glu
            115                           120                           125

Ser Gly Gly Gly Leu Val Gln Pro Gly Gly Ser Leu Arg Leu Ser Cys
      130                           135                           140

Ala Ala Ser Gly Tyr Ser Phe Thr Gly Tyr Thr Met Asn Trp Val Arg
145                          150                            155
160
Gln Ala Pro Gly Lys Cys Leu Glu Trp Val Ala Leu Ile Asn Pro Tyr
                        165                           170
175
Lys Gly Val Thr Thr Tyr Ala Asp Ser Val Lys Gly Arg Phe Thr Ile
                  180                           185                 190
```

Ser Val Asp Lys Ser Lys Asn Thr Ala Tyr Leu Gln Met Asn Ser Leu
195 200 205

Arg Ala Glu Asp Thr Ala Val Tyr Tyr Cys Ala Arg Ser Gly Tyr Tyr
210 215 220

Gly Asp Ser Asp Trp Tyr Phe Asp Val Trp Gly Gln Gly Thr Leu Val
225 230 235
240
Thr Val Ser Ser

<210> 18
<211> 111
<212> PRT
<213> Artificial Sequence

<220>
<223> Laboratory synthesis

<400> 18
Asp Ile Gln Leu Thr Gln Ser Pro Ala Ser Leu Ala Val Ser Leu Gly
1 5 10
15
Gln Arg Ala Thr Ile Ser Cys Lys Ala Ser Gln Ser Val Asp Tyr Asp
20 25 30

Gly Asp Ser Tyr Leu Asn Trp Tyr Gln Gln Ile Pro Gly Gln Pro Pro
35 40 45

Lys Leu Leu Ile Tyr Asp Ala Ser Asn Leu Val Ser Gly Ile Pro Pro
50 55 60

Arg Phe Ser Gly Ser Gly Ser Gly Thr Asp Phe Thr Leu Asn Ile His
65 70 75
80
Pro Val Glu Lys Val Asp Ala Ala Thr Tyr His Cys Gln Gln Ser Thr
85 90
95
Glu Asp Pro Trp Thr Phe Gly Gly Gly Thr Lys Leu Glu Ile Lys
100 105 110

<210> 19
<211> 124
<212> PRT
<213> Artificial Sequence

<220>
<223> Laboratory synthesis

<400> 19
Gln Val Gln Leu Gln Gln Ser Gly Ala Glu Leu Val Arg Pro Gly Ser
1 5 10
15
Ser Val Lys Ile Ser Cys Lys Ala Ser Gly Tyr Ala Phe Ser Ser Tyr
20 25 30

Trp Met Asn Trp Val Lys Gln Arg Pro Gly Gln Gly Leu Glu Trp Ile
35 40 45

Gly Gln Ile Trp Pro Gly Asp Gly Asp Thr Asn Tyr Asn Gly Lys Phe
50 55 60

Lys Gly Lys Ala Thr Leu Thr Ala Asp Glu Ser Ser Ser Thr Ala Tyr
65 70 75
80
Met Gln Leu Ser Ser Leu Ala Ser Glu Asp Ser Ala Val Tyr Phe Cys
85 90
95
Ala Arg Arg Glu Thr Thr Thr Val Gly Arg Tyr Tyr Tyr Ala Met Asp
100 105 110

Tyr Trp Gly Gln Gly Thr Thr Val Thr Val Ser Ser
115 120

<210> 20
<211> 451
<212> PRT
<213> Artificial Sequence

<220>
<223> Laboratory synthesis

<400> 20
Gln Val Gln Leu Gln Gln Ser Gly Ala Glu Leu Val Arg Pro Gly Ser
1 5 10
15
Ser Val Lys Ile Ser Cys Lys Ala Ser Gly Tyr Ala Phe Ser Ser Tyr
20 25 30

Trp Met Asn Trp Val Lys Gln Arg Pro Gly Gln Gly Leu Glu Trp Ile
35 40 45

Gly Gln Ile Trp Pro Gly Asp Gly Asp Thr Asn Tyr Asn Gly Lys Phe
50 55 60
Lys Gly Lys Ala Thr Leu Thr Ala Asp Glu Ser Ser Ser Thr Ala Tyr
65 70 75
80
Met Gln Leu Ser Ser Leu Ala Ser Glu Asp Ser Ala Val Tyr Phe Cys
85 90
95
Ala Arg Arg Glu Thr Thr Thr Val Gly Arg Tyr Tyr Tyr Ala Met Asp
100 105 110

Tyr Trp Gly Gln Gly Thr Thr Val Thr Val Ser Ser Ala Ser Thr Lys
115 120 125

Gly Pro Ser Val Phe Pro Leu Ala Pro Cys Ser Arg Ser Thr Ser Glu
130 135 140

Ser Thr Ala Ala Leu Gly Cys Leu Val Lys Asp Tyr Phe Pro Glu Pro
145 150 155
160
Val Thr Val Ser Trp Asn Ser Gly Ala Leu Thr Ser Gly Val His Thr
165 170
175
Phe Pro Ala Val Leu Gln Ser Ser Gly Leu Tyr Ser Leu Ser Ser Val
180 185
190
Val Thr Val Pro Ser Ser Ser Leu Gly Thr Lys Thr Tyr Thr Cys Asn
195 200 205

Val Asp His Lys Pro Ser Asn Thr Lys Val Asp Lys Arg Val Glu Ser
210 215 220

Lys Tyr Gly Pro Pro Cys Pro Pro Cys Pro Ala Pro Glu Phe Leu Gly

225 230 235
240
Gly Pro Ser Val Phe Leu Phe Pro Pro Lys Pro Lys Asp Thr Leu Met
245 250
255
Ile Ser Arg Thr Pro Glu Val Thr Cys Val Val Val Asp Val Ser Gln
260 265 270

Glu Asp Pro Glu Val Gln Phe Asn Trp Tyr Val Asp Gly Val Glu Val
275 280 285

His Asn Ala Lys Thr Lys Pro Arg Glu Glu Gln Phe Asn Ser Thr Tyr
290 295 300

Arg Val Val Ser Val Leu Thr Val Leu His Gln Asp Trp Leu Asn Gly
305 310 315
320
Lys Glu Tyr Lys Cys Lys Val Ser Asn Lys Gly Leu Pro Ser Ser Ile
325 330
335
Glu Lys Thr Ile Ser Lys Ala Lys Gly Gln Pro Arg Glu Pro Gln Val
340 345 350

Tyr Thr Leu Pro Pro Ser Gln Glu Glu Met Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395
400
Val Leu Asp Ser Asp Gly Ser Phe Phe Leu Tyr Ser Arg Leu Thr Val
405 410
415
Asp Lys Ser Arg Trp Gln Glu Gly Asn Val Phe Ser Cys Ser Val Met
420 425
430
His Glu Ala Leu His Asn His Tyr Thr Gln Lys Ser Leu Ser Leu Ser
435 440 445

Leu Gly Lys
450

<210> 21
<211> 1353
<212> DNA
<213> Artificial Sequence

<220>
<223> Laboratory synthesis

<400> 21
caagttcaac ttcagcagtc tggcgccgaa ctcgtgcggc ctggatctag cgtgaagatc 60
agctgtaaag ccagcggcta cgccttcagc agctactgga tgaactgggt caagcagagg 120
cctggacagg gcctcgaatg gatcggacaa atttggcctg gcgacggcga caccaactac 180
aacggcaagt tcaagggcaa agccacactg accgccgacg agtctagcag cacagcctac 240
atgcagctga gcagcctggc ctctgaagat agcgccgtgt acttctgcgc cagacgggaa 300
acaaccaccg tgggcagata ttactacgcc atggactact ggggccaggg caccacagtg 360
acagttagct ctgcgtcgac caagggcccc agcgtgttcc ccctggcccc ttgcagcaga 420
agcaccagcg agagcacagc cgccctgggc tgcctggtga aggactactt ccccgagccc 480
gtgaccgtgt cctggaacag cggcgctctg accagcggcg tgcatacctt ccccgccgtg 540
ctccagagca gcggactgta ctccctgagc agcgtggtga cggtgccttc cagcagcctg 600

```
ggcaccaaga cctacacttg caacgtggac cacaagccca gcaacaccaa ggtggacaag     660
agagtggaga gcaagtacgg ccctccctgc ccccttgcc ctgcccccga gttcctgggc      720
ggacctagcg tgttcctgtt cccccccaag cccaaggaca ccctgatgat cagcagaacc     780
cccgaggtga cgtgcgtggt ggtggacgtg tcccaggagg accccgaggt ccagtttaat     840
tggtacgtgg acggcgtgga agtgcataac gccaagacca agcccagaga ggagcagttc     900
aacagcacct acagagtggt gtccgtgctg accgtgctgc accaggactg gctgaacggc     960
aaggaataca agtgcaaggt ctccaacaag ggcctgccta gcagcatcga gaagaccatc     1020
agcaaggcca agggccagcc acgggagccc caggtctaca ccctgccacc tagccaagag     1080
gagatgacca agaaccaggt gtccctgacc tgtctggtga aaggcttcta tcccagcgat     1140
atcgccgtgg agtgggagag caacggccag cccgagaaca actacaagac caccccccct     1200
gtgctggaca gcgacggcag cttcttcctg tactccagac tgaccgtgga caagtccaga     1260
tggcaggagg gcaacgtctt cagctgctcc gtgatgcacg aggccctgca caaccactac     1320
acccagaagt ccctgagcct gagcctgggc aag     1353
```

## Description

### Cross Reference

The present application claims priority to Chinese patent application No. 201910209330.4 entitled "Bispecific antibody, preparation method thereof and application thereof', filed on March 19, 2019, the entire disclosure of which is incorporated herein by reference in its entirety.

### Technical Field

The present invention relates to the technical fields of biotechnology and immunology, specifically, to a bispecific antibody that binds CD19 and CD3, and preparation method thereof and use thereof.

### Background Art

Antibody drugs are biomacromolecule drugs prepared by antibody engineering technologies with cell engineering technology and genetic engineering technology as the main body, and have the advantages of high specificity, uniform properties, and customized preparation for specific targets. Monoclonal antibodies are mainly used clinically in the following three aspects: tumor treatment, immune disease treatment and anti-infection treatment, wherein, tumor treatment is currently the most widely used field of monoclonal antibodies. Currently, among the current monoclonal antibody products that have entered clinical trials and are on the market, products used for tumor treatment account for approximately 50%. Monoclonal antibody treatment of tumors is an immunotherapy aimed at specific targets of diseased cells to stimulate the immune system to kill target cells. To enhance the effector function of antibodies, especially the effect of killing tumor cells, people have tried a variety of methods to modify antibody molecules. A bispecific antibody is one of the development directions to improve the therapeutic effect of antibodies and has become a hot spot in the field of antibody engineering research.

A bispecific antibody (BsAb) is an artificial antibody that can specifically recognize and bind to two different antigens or epitopes. If the two antigens are located on surface of different cells, the bispecific antibody can set up a bridge between the two antigen molecules, thereby forming cross-links between cells and mediating the cells to produce directed effector functions. BsAb has broad application prospects in biomedicine, especially in tumor immunotherapy. Bispecific antibodies (immune double antibodies) used for immunotherapy are artificial antibodies containing two specific antigen binding sites that bind to cell receptor antigens, and they can set up a bridge between diseased cells (target cells) and functional cells (immune cells), to stimulate a directed immune response. The killing of tumor cells by BsAb-mediated immune cells (such as T cells, NK cells and the like) is currently a hot spot in the application research of immunotherapy. The mechanism of action is that BsAb can simultaneously bind to tumor-related antigens and target molecules on immune effector cells, and directly leads to the specific killing of tumor cells by immune effector cells while activating immune cells.

Bispecific antibodies can be obtained through a variety of ways, and the preparation methods thereof mainly include chemical coupling method, hybrid-hybridoma method and genetic engineering antibody preparation method. The chemical coupling method comprises preparing a bispecific monoclonal antibody (which is the earliest bispecific monoclonal antibody) by chemically coupling two different monoclonal antibodies together. The hybrid-hybridoma method comprises producing a bispecific monoclonal antibody by means of double hybridoma fusion method or ternary hybridomas. These cell hybridomas or ternary hybridomas are obtained by the fusion of established hybridomas, or the fusion of established hybridomas with lymphocytes from mice and can only be used to produce murine bispecific antibodies, and thus, the application thereof is greatly limited. With the rapid development of molecular biology technology, there have emerged a variety of construction modes of genetically engineered humanized or fully human bispecific antibodies, mainly including four classes of bispecific miniantibodies, double-chain diabodies, single-chain bivalent antibodies, and multivalent bispecific antibodies. At present, several genetically engineered bispecific antibody drugs have entered clinical trials in the world and have shown good application prospects.

The CD3 molecule on the surface of a T cell consists of 4 subunits: $\delta$, $\varepsilon$, $\gamma$ and $\zeta$, the molecular masses thereof are 18.9 kDa, 23.1 kDa, 20.5 kDa and 18.7 kDa, respectively, and the lengths thereof are 171, 207, 182, and 164 amino acid residues, respectively. They form 6 peptide chains together, which are often tightly bound to a T cell receptor (TCR) to form a TCR-CD3 complex containing 8 peptide chains, and the schematic diagram of the structure thereof is shown in Figure 1. The complex has the functions of T cell activation, signal transduction and stabilization of the TCR structure. The cytoplasmic segment of CD3 contains immunoreceptor tyrosine-based activation motif (ITAM). TCR recognizes and binds to antigen peptides presented by MHC (major histo-compatibility complex) molecules, rendering the tyrosine residues in the conserved sequence of ITAM of CD3 being phosphorylated by tyrosine protein kinase p561ck in T cells, and then other tyrosine protein kinases (such as ZAP-70) containing SH2 (Scr homology 2) domain being recruited. The phosphorylation of ITAM together with its binding to ZAP-70 is one of the important biochemical reactions in the early stages of the signal transduction process of T cell activation. Therefore, the function of CD3 molecule is to transduce the activation signal generated by recognition of a TCR antigen.

CD19, also known as B4 or Leu-12, belongs to the immunoglobulin (Ig) superfamily, which has a molecular weight of 95 kDa, located on the short arm of chromosome 16, contains 15 exons, and encodes a type I transmembrane glycoprotein of 556 amino acids. When immunoglobulin genes are recombined, CD19 is first expressed in late progenitor B cells and early pre-B cells. CD19 is highly expressed throughout the development and maturation of B cells, and until the B cells differentiate into plasma cells, the expression level is downregulated and its expression in mature B cells is three times that of immature cells.

CD19 establishes B cell signal threshold by simultaneously regulating B cell receptor (BCR) dependent and independent signals, and plays an important regulatory role in the development, proliferation, and differentiation of B cells. As a main component of the surface multi-molecular complex of mature B cells, CD19 forms a complex together with receptors CD21 (CD2), CD81 (TAPA-1) and CD225, and reduces the threshold of antigen concentration required for triggering B cell division and differentiation by regulating endogenous and receptor-induced signals. As a chaperone protein, CD81 provides a molecular docking site for signal transduction pathways and regulates the expression of CD19. CD19 activates protein tyrosine kinase (PTK) by recruiting and amplifying the activation of Src family protein tyrosine kinases and activates BCR signals. Meanwhile, when BCR signals are activated, CD19 can also enhance BCR signals and promote proliferation of B cells by activating PI3K and downstream Akt kinase.

CD19 is expressed both in normal and malignant B lymphocytes and is regarded as one of the most reliable surface markers covering a long period of time during the development of B cells. In normal lymphoid tissues, CD19 is expressed in pre-B cells, B cells and follicular dendritic cells, mantle cells, and dendritic cells in the inter-follicular T cell area. In addition, CD19 can be detected in plasma cells isolated from human tissues through flow cytometry. CD19 is expressed in B lymphocytomas, including B lymphocytic lymphoma, small lymphocytic lymphoma, mantle cell lymphoma, follicular lymphoma, Burkitt lymphoma, and marginal zone lymphoma. Therefore, CD19 has become a specific molecular target for the treatment of B-cell malignant tumors. In recent years, immunotherapy strategies targeting CD19 have been extensively developed in preclinical and clinical studies, including monoclonal antibodies, bispecific antibodies, and chimeric antigen receptor modified T cells (CAR-T), and clinical effects significantly better than conventional small molecule chemotherapy have been achieved, thereby promoting the progress of immunotherapy.

Adoptive immunotherapy for tumors is to inject autologous or allogeneic immunocompetent cells expanded *in vitro* into a patient to directly kill tumor cells, regulate and enhance the body's immune function, mainly including immunotherapy with LAK cells, TIL cells, activated T lymphocytes and CIK cells. However, immunotherapy can only remove a small number of scattered tumor cells, and has limited efficacy for advanced solid tumors. Therefore, immunotherapy is often used as an adjuvant therapy in combination with conventional methods such as surgery, chemotherapy, radiotherapy and the like. After many tumor cells are firstly cleaned up by conventional methods, then immunotherapy is used to remove remaining tumor cells, and thus the effect of comprehensive tumor treatment can be improved. As a new method in the comprehensive treatment of tumors, adoptive immunotherapy has been widely combined with conventional surgical treatment, radiotherapy, chemotherapy and other cell and molecular therapies, and has broad application prospects in the treatment of various tumors. However, in combination with bispecific antibodies, the ideal adoptive immunotherapy for tumors should be: the bispecific antibody has one end bound to a surface antigen (such as CD3) of immune cells, which is introduced into the body together with the immune cells, while the other end of the bispecific antibody can be well bound to a surface antigen of tumor cells; and in this way, the bispecific antibody can build a bridge between tumor cells and immune cells in the body, so that the immune cells are concentrated around the tumor cells, thereby killing the tumor cells. The metastasis and spread of tumor cells can be effectively solved by this method, which overcomes the disadvantages such as "incomplete, easy to metastasize, and severe side effects" after the three traditional treatments of surgery, radiotherapy and chemotherapy. Therefore, it is of great significance to develop a highly efficient bispecific antibody that binds tumor cells and immune cells.

## Summary of the Invention

In order to solve the technical problems in the prior art, the purpose of the present invention is to provide a bispecific antibody that binds CD19 and CD3, has a specific targeting effect, and can efficiently stimulate a directed immune response, and a preparation method therefor and use thereof.

In order to achieve the above purpose, the technical solution of the present invention is as follows: by designing and screening of the molecular structure of a bispecific antibody that binds CD19 and CD3, the present invention creatively finds that as compared with corresponding monoclonal antibody and bispecific antibodies with other structures, a bispecific antibody with the following symmetrical structure can better retain the specific binding ability of the original antibody, and meanwhile, has the biological functions of two monoclonal antibodies, and has obvious advantages in terms of production process and medicinal properties: a bispecific antibody comprises (a) a monoclonal antibody unit which consists of two complete light chain-heavy chain pairs, and (b) a single-chain antibody unit comprising two identical single-chain antibodies containing a heavy chain variable region and a light chain variable region, wherein the single-chain antibody unit has the capacity of specifically binding to the surface antigen CD3 of immune cells, the monoclonal antibody unit has the capacity of specifically binding to the surface antigen CD19 of tumor cells, and the single-chain antibody unit is linked to the N-end or C-end of the monoclonal antibody unit through a linker peptide. The present invention has developed a bispecific antibody with the above-mentioned antibody molecular structure that binds CD19 and CD3. This bispecific antibody has a specific targeting effect and can efficiently stimulate a directed immune response and kill tumor cells.

Specifically, firstly, the present invention provides a bispecific antibody, the bispecific antibody comprises (a) a monoclonal antibody unit and (b) a single-chain antibody unit; the monoclonal antibody unit consists of two complete light chain-heavy chain pairs, and can specifically bind to CD19; the single-chain antibody unit comprises two single-chain antibodies (ScFv), and the single-chain antibody comprises a heavy chain variable region and a light chain variable region, and can specifically bind to CD3. The bispecific antibody has a symmetric structure formed by linkage in any one of the following modes:

(1) N-ends of the two single-chain antibodies are respectively linked to C-ends of two heavy chains of the monoclonal antibody through a linker peptide; and
(2) C-ends of the two single-chain antibodies are respectively linked to N-ends of two heavy chains of the monoclonal antibody through a linker peptide.

Preferably, the amino acid sequence of the linker peptide is (GGGGX)n, wherein X is Gly or Ser, and n is a natural number selected from 1 to 4 (that is, 1, 2, 3 or 4). When the linker peptide with the above sequence is used, the bispecific antibody can better perform the antigen-binding function.

As a preferred embodiment of the present invention, the amino acid sequence of the linker peptide is represented by SEQ ID NO. 13.

Preferably, the light chain sequence of the single-chain antibody is represented by SEQ ID NO. 5 or represented by SEQ ID NO. 9.

The heavy chain sequence of the single-chain antibody is represented by SEQ ID NO. 6 or represented by SEQ ID NO. 10.

Both the light chain and the heavy chain of the single-chain antibody can specifically bind to the surface antigen CD3 of immune cells.

In the present invention, the single-chain antibodies are expressed as fusion peptides. Through the specific design of antibody structure and sequence, it is found that when the single-chain antibody and the monoclonal antibody are linked in different ways, the stability of the antibody structure and the binding to two antigens can be better improved by adopting specific fusion peptide sequences of the single-chain antibody, respectively.

For the single-chain antibody unit of the bispecific antibody, preferably, the light chain and the heavy chain of the single-chain antibody constitute a fusion peptide, and the sequence of the fusion peptide is any one of the follows:

(1) when N-ends of the two single-chain antibodies are respectively linked to C-ends of the two heavy chains of the monoclonal antibody through a linker peptide, the sequence of the fusion peptide is represented by SEQ ID NO. 17; and
(2) when C-ends of the two single-chain antibodies are respectively linked to N-ends of two heavy chains of the monoclonal antibody through a linker peptide, the sequence of the fusion peptide is represented by SEQ ID NO. 16.

For the monoclonal antibody unit of the bispecific antibody, preferably, the sequence of the light chain variable region of the monoclonal antibody is represented by SEQ ID NO. 18, or is the amino acid sequence of a polypeptide with the same function which is obtained by subjecting the amino acid sequence represented by SEQ ID NO. 18 to substitution, deletion or insertion of one or more amino acids.

The sequence of the heavy chain variable region of the monoclonal antibody is represented by SEQ ID NO. 19, or is the amino acid sequence of a polypeptide with the same function which is obtained by subjecting the amino acid sequence represented by SEQ ID NO. 19 to substitution, deletion or insertion of one or more amino acids. In the present invention, the bispecific antibody may be a murine antibody, a humanized antibody, a chimeric antibody or a recombinant antibody.

As an embodiment of the present invention, the light chain and the heavy chain of the monoclonal antibody are connected by a disulfide bond. The Fc fragment of the monoclonal antibody is a Fc fragment of a human or humanized antibody.

Preferably, the human or humanized antibody comprises one of IgG1 antibody, IgG2 antibody, IgG3 antibody, and IgG4 antibody.

As a preferred embodiment of the present invention, the Fc fragment of the monoclonal antibody is a Fc fragment of a human or humanized IgG4 antibody.

As a preferred embodiment of the present invention, a full-length sequence of the light chain of the monoclonal antibody is represented by SEQ ID NO. 3; and a full-length sequence of the heavy chain of the monoclonal antibody is represented by SEQ ID NO. 1 or SEQ ID NO. 20.

In the present invention, the above-mentioned "amino acid sequence of a protein with the same function which is obtained by substitution, deletion or insertion of one or more amino acids" refers to a sequence which is different from the shown sequence at one or more amino acid residues but the resulting molecule can retain the biological activity, and it can be a "conservatively modified variant" or obtained by modification through "conservative amino acid substitution". "Conservatively modified variant" or "conservative amino acid substitution" refers to an amino acid substitution known to a person skilled in the art which generally does not change the biological activity of the obtained molecule. It is acknowledged by a person skilled in the art that the substitution of a single amino acid in the nonessential region of a polypeptide basically does not change the biological activity. Exemplary substitutions are preferably carried out in accordance with the substitutions shown below:

Table 1: Exemplary conservative amino acid substitution table

| Original residues | Conservative substitution |
|---|---|
| Ala (A) | Gly, Ser |
| Arg (R) | Lys, His |
| Asn (N) | Gln, His |
| Asp (D) | Glu, Asn |
| Cys (C) | Ser, Ala |
| Gln (Q) | Asn |
| Glu (E) | Asp, Gln |
| Gly (G) | Ala |
| His (H) | Asn, Gln |
| Ile (I) | Leu, Val |
| Lys (K) | Arg, His |
| Met (M) | Leu, Ile, Tyr |
| Phe (F) | Tyr, Met, Leu |
| Pro (P) | Ala |
| Ser (S) | Thr |
| Thr (T) | Ser |
| Trp (W) | Tyr, Phe |
| Tyr (Y) | Trp, Phe |
| Val (V) | Ile, Leu |

As an example of the above-mentioned bispecific antibody, the present invention provides a bispecific antibody against human CD3 and CD19. Among the structures having heavy chain variable region and light chain variable region of the above-mentioned single-chain antibody and heavy chain and light chain of the monoclonal antibody and sequences, two bispecific antibodies binding to CD3 and CD19 that retain the biological function of the corresponding monoclonal antibody to the greatest extent and have obvious advantages in terms of production process and medicinal properties are obtained by screening in the present invention. The structures and sequences of the two bispecific antibodies are as follows:

(1) The sequence of the light chain and heavy chain fusion peptide of the single-chain antibody is represented by SEQ ID NO. 16, the light chain sequence of the monoclonal antibody is represented by SEQ ID NO. 3, and the heavy chain sequence of the monoclonal antibody is represented by SEQ ID NO. 1. The antibody structure is a symmetric structure in which the C-ends of two single-chain antibody fusion peptides are respectively linked to the N-ends of the two heavy chains of the monoclonal antibody through a linker peptide represented by SEQ ID NO. 13 (as shown in Figure 2 A).

(2) The sequence of the light chain and heavy chain fusion peptide of the single-chain antibody is represented by SEQ ID NO. 17, the light chain sequence of the monoclonal antibody is represented by SEQ ID NO. 3, and the heavy chain sequence of the monoclonal antibody is represented by SEQ ID NO. 20. The antibody structure is a symmetric structure in which the N-ends of two single-chain antibody fusion peptides are respectively linked to the C-ends of the two heavy chains of the monoclonal antibody through the linker peptide represented by SEQ ID NO. 13 (as shown in Figure 2 B).

Based on the above-mentioned amino acid sequences of the bispecific antibody, the present invention also provides a gene encoding the bispecific antibody.

According to the codon coding rules and the degeneracy and preference of the codon, a person skilled in the art can design the coding gene according to the above-mentioned amino acid sequences of the bispecific antibody.

As a preferred embodiment of the present invention, a gene sequence coding the full-length light chain of the monoclonal antibody is represented by SEQ ID NO. 4.

As a preferred embodiment of the present invention, a gene sequence coding the full-length heavy chain of the monoclonal antibody is represented by SEQ ID NO. 2 or represented by SEQ ID NO. 21.

As a preferred embodiment of the present invention, when the C-ends of the two single-chain antibodies are respectively linked to the N-ends of the two heavy chains of the monoclonal antibody through a linker peptide, a gene sequence coding the single-chain antibody is represented by SEQ ID NO. 14; and when the N-ends of the two single-chain antibodies are respectively linked to the C-ends of the two heavy chains of the monoclonal antibody through a linker peptide, a gene sequence coding the single-chain antibody is represented by SEQ ID NO. 15.

The above-mentioned gene sequences can be combined to express the bispecific antibody or can be respectively combined with other coding gene sequences of the remaining units of the bispecific antibody to express the bispecific antibody.

Further, the present invention also provides a biological material comprising the above-mentioned gene.

In the present invention, the biological material comprises a recombinant DNA, an expression cassette, a vector, a host cell, an engineered bacterium or cell line.

The present invention also provides a preparation method of the bispecific antibody, comprising: constructing an expression vector containing coding genes of the single-chain antibody and the monoclonal antibody; introducing the expression vector into a host cell to obtain a host cell stably expressing the bispecific antibody; culturing the host cell, and obtaining the bispecific antibody by separation and purification.

When preparing the bispecific antibody, a person skilled in the art can select the host cell, expression vector, method for introducing the expression vector into the host cell and separation and purification method of the antibody that are conventional in the art as needed.

As an embodiment of the present invention, the host cell is CHO-K1 cell.

As an embodiment of the present invention, the expression vector is pG4HK

The construction of the expression vector can use conventional methods in the art. As a preferred embodiment of the present invention, the construction method of the expression vector comprises: linking the light chain coding gene of the anti-CD 19 monoclonal antibody to an expression vector pG4HK by double enzyme digestion with SalI and BsiWI to obtain an expression vector of anti-CD 19 light chain named as pG4HK19VL; and linking the fusion fragment of the anti-CD3 single-chain antibody coding gene and the heavy chain gene of the anti-CD19 monoclonal antibody to a vector pG4HK19VL by double enzyme digestion with Hind III and BstEII to obtain a bispecific antibody expression vector.

The separation and purification can be performed by antibody separation and purification method commonly used in the art.

As an embodiment of the present invention, the separation and purification comprises the following steps:

(1) separating all antibodies with Fc domain from a culture supernatant through a recombinant rProtein A affinity chromatography column;
(2) separating a bispecific antibody from by-products by anion exchange Q-Sepharose column chromatography; and
(3) purifying the bispecific antibody by molecular sieve chromatography.

Based on the above-mentioned bispecific antibody, the present invention also provides a pharmaceutical composition comprising the bispecific antibody of the present invention.

Preferably, the pharmaceutical composition also comprises other pharmaceutically acceptable active ingredients or adjuvants.

Further, the present invention provides any one of the following uses of the bispecific antibody or the coding gene of the bispecific antibody or the biological material comprising the coding gene:

(1) use in the preparation of a drug for prevention or treatment of CD19-expressing B cell-related diseases;
(2) use in the preparation of a drug for prevention or treatment of a disease with CD 19 as a target;
(3) use in the preparation of a drug for killing CD19-expressing cells; and
(4) use in the preparation of a detection reagent for CD19 and/or CD3.

In the present invention, the CD19-expressing B cell-related diseases include but are not limited to B cell-related tumors and autoimmune diseases caused by B cells.
The B cell-related tumors are not limited to B-lymphocytoma and B-lineage leukemia.
The beneficial effects of the present invention are as follows:
By genetic engineering and antibody engineering methods, the present invention constructs a bispecific antibody that comprises a single-chain antibody and a complete monoclonal antibody structure and binds to CD19 and CD3. The bispecific antibody fusion protein retains a complete monoclonal antibody structure, and has a highly stable symmetrical structure, better retains the biological functions of an anti-CD3 single-chain antibody and an anti-CD 19 monoclonal antibody, realizes a bispecific antibody molecule simultaneously having excellent biological functions of anti-CD 19 and anti-CD3 monoclonal antibodies, which can build a bridge between tumor cells and immune effector cells, effectively activate immune effector cells and directed immune responses, significantly enhance the efficacy of immune cells to kill tumor cells, and minimize the ADCC effect, with high safety. In addition, because the bispecific antibody provided by the present invention has a feature of completely symmetrical structure, when expressed in the host, no protein isomers of other structures will be produced, thus the difficulty of extraction and purification process is greatly reduced. The bispecific antibody has the advantages of simple preparation and high yield and has broad application prospects in tumor immunotherapy.

## Brief Description of the Drawings

Figure 1 is a schematic diagram of the structure of the cell surface antigen CD3 molecule in the background of the present invention.
Figure 2 is a schematic diagram of the molecular structures of two bispecific antibodies YK001 and YK002 obtained through screening in Example 1 of the present invention, wherein A represents the bispecific antibody YK001; and B represents the bispecific antibody YK002.
Figure 3 is the SDS-PAGE electrophoresis diagram of the bispecific antibodies YK001 and YK002 in Example 2 of the present invention, wherein A and C represent reduced SDS-PAGE electrophoresis detection; B and D represent non-reduced SDS-PAGE electrophoresis detection; A and B represent SDS-PAGE electrophoresis results of YK001 bispecific antibody; C and D represent SDS-PAGE electrophoresis results of YK002 bispecific antibody; M represents protein molecular weight marker, and lane 1 represents the target protein.
Figure 4 shows HPLC-SEC purity peak graphs of bispecific antibodies YK001 and YK002 in Example 2 of the present invention, wherein A represents the bispecific antibody YK001; and B represents the bispecific antibody YK002.
Figure 5 shows the binding efficiency of bispecific antibodies YK001 and YK002 with Raji cells determined based on flow cytometry in Example 3 of the present invention, wherein A represents the negative control NC; B represents the bispecific antibody YK001; C represents the positive control antibody (PC) Anti-CD 19; D represents the negative control NC; E represents the bispecific antibody YK002; and F represents the positive control antibody (PC) Anti-CD 19.
Figure 6 shows the binding efficiency of bispecific antibodies YK001 and YK002 with T cells determined based on flow cytometry in Example 3 of the present invention, wherein A represents the negative control NC; B represents the bispecific antibody YK001; C represents the bispecific antibody YK002, and D represents the positive control (PC) Anti-CD3.
Figure 7 is a diagram showing the results in Example 4 of the present invention that the bispecific antibodies YK001 and YK002 effectively mediated PBMC cells to kill Raji tumor cells, wherein (▼) represents the bispecific antibody YK001, (▽) represents the bispecific antibody YK002, (■) represents Anti-CD19 monoclonal antibody, (◇) represents irrelevant control 0527×CD3 bispecific antibody (Her2×CD3 bispecific antibody), and (●) represents Anti-CD3 monoclonal antibody.

## Specific Modes for Carrying Out the Embodiments

The preferred embodiments of the present invention will be described in detail below in conjunction with Examples. It should be understood that the following Examples are given for illustrative purposes only and are not intended to limit the scope of the present invention. A person skilled in the art can make various modifications and alternatives to the present invention without departing from the aim and spirit of the present invention.

The experimental methods used in the following examples are conventional methods unless otherwise specified.

The materials and reagents used in the following Examples can be obtained from commercial sources unless otherwise specified.

**Example 1: Design of the structure and sequence of CD19×CD3 bispecific antibody**

In the present Example, the tumor cell surface antigen CD19 and the immune cell surface antigen CD3 were used as targets to design a bispecific antibody.

Combined with protein structure design software and a lot of artificial experimental screening, a variety of CD19 and CD3 binding bispecific antibody structures were screened in the present invention for bispecific antibody structures with symmetrical structures comprising a single-chain antibody unit and a monoclonal antibody unit, wherein the anti-CD 19 monoclonal antibody unit is an IgG antibody, and comprises two complete light chain-heavy chain pairs (i.e., containing complete Fab and Fc domains, and the heavy chain and the light chain are connected by a disulfide bond), the anti-CD3 single-chain antibody unit comprises two single-chain antibodies (ScFv), each single-chain antibody contains a heavy chain variable region domain and a light chain variable region domain, and the heavy chain variable region and the light chain variable region are constructed as a fusion peptide through a linker peptide. The single-chain antibody and the monoclonal antibody are linked by a linker peptide. For the linkage modes between the single-chain antibody and the monoclonal antibody, two different linkage methods were designed to obtain two bispecific antibodies with different symmetric structures:

(1) The C-ends of the anti-CD3 single-chain antibody were linked to the N-ends of the heavy chain of the anti-CD 19 monoclonal antibody through a linker peptide of GGGGSGGGGSGGGGS (represented by SEQ ID NO. 13) to obtain a bispecific antibody YK001 (with the structure schematic diagram as shown in Figure 2 A); and
(2) The N-ends of the anti-CD3 single-chain antibody were linked to the C-ends of the heavy chain of the anti-CD 19 monoclonal antibody through a linker peptide of GGGGSGGGGSGGGGS (represented by SEQ ID NO. 13) to obtain a bispecific antibody YK002 (with the structure schematic diagram as shown in Figure 2 B).

The amino acid sequence of each domain of the above-mentioned bispecific antibody is as follows:

The amino acid sequence of the heavy chain variable region of the anti-CD 19 monoclonal antibody of YK001 is represented by SEQ ID NO. 19, and the amino acid sequence of the full-length heavy chain is represented by SEQ ID NO. 1.
The amino acid sequence of the heavy chain variable region of the anti-CD 19 monoclonal antibody of YK002 is represented by SEQ ID NO. 19, and the amino acid sequence of the full-length heavy chain is represented by SEQ ID NO. 20.
The amino acid sequence of the light chain variable region of the anti-CD 19 monoclonal antibody is represented by SEQ ID NO. 18, and the amino acid sequence of the full-length light chain is represented by SEQ ID NO. 3 (same for YK001 and YK002).
The amino acid sequence of the anti-CD3 single-chain antibody in YK001 is represented by SEQ ID NO. 16.
The amino acid sequence of the anti-CD3 single-chain antibody in YK002 is represented by SEQ ID NO. 17.

**Example 2: Preparation of a CD19×CD3 bispecific antibody**

1. Design and synthesis of coding genes of the bispecific antibody

According to the amino acid sequences of the two bispecific antibodies YK001 and YK002 obtained by the design and screening in Example 1, and the codon preference of the host cell, the coding genes of the bispecific antibodies were designed, with the specific sequences as follows:

the nucleotide sequence coding the heavy chain of the anti-CD 19 monoclonal antibody of YK001 was represented by SEQ ID NO. 2;
the nucleotide sequence coding the heavy chain of the anti-CD 19 monoclonal antibody of YK002 was represented by SEQ ID NO. 21;
the nucleotide sequence coding the light chain of the anti-CD 19 monoclonal antibody was represented by SEQ ID NO. 4 (same for YK001 and YK002);

the nucleotide sequence coding the anti-CD3 single-chain antibody in YK001 was represented by SEQ ID NO. 14; and the nucleotide sequence coding the anti-CD3 single-chain antibody in YK002 was represented by SEQ ID NO. 15.

In order to facilitate the construction of expression vectors, the gene fragment coding the light chain of the anti-CD19 monoclonal antibody (same for YK001 and YK002) and the fusion fragment of the coding gene of the anti-CD3 single-chain antibody and the coding gene of the heavy chain of the anti-CD19 monoclonal antibody (YK001, i.e., the C-end of the anti-CD3 single-chain antibody is linked to the N-end of the heavy chain of the anti-CD19 monoclonal antibody) and the fusion fragment of the coding gene of the heavy chain of the anti-CD19 monoclonal antibody and the coding gene of the anti-CD3 single-chain antibody (YK002, i.e., the N-end of the anti-CD3 single-chain antibody is connected to the C-end of the heavy chain of the anti-CD19 monoclonal antibody) were synthesized.

2. Construction of bispecific antibody expression vectors

(1) The coding gene of the light chain of the anti-CD19 monoclonal antibody was linked to the expression vector pG4HK by double enzyme digestion with SalI and BsiWI to obtain the expression vector of the light chain of the anti-CD 19 monoclonal antibody named as pG4HK19VL.
(2) The fusion fragment of the coding gene of the anti-CD3 single-chain antibody and the coding gene of the heavy chain of the anti-CD 19 monoclonal antibody was linked to the vector pG4HK19VL by double enzyme digestion with Hind III and BstE II to obtain the YK001 bispecific antibody expression vector name as pG4HK-YK001.
(3) The fusion fragment of the coding gene of the heavy chain of the anti-CD19 monoclonal antibody and the coding gene of the anti-CD3 single-chain antibody coding gene was linked to the vector pG4HK19VL by double enzyme digestion with Hind III and BstE II to obtain the YK002 bispecific antibody expression vector named as pG4HK-YK002.

3. Expression of bispecific antibodies

(1) Plasmid was subject to large-scale extraction with an endotoxin-free large-scale extraction kit (Qiagen, 4991083), and the specific operation was carried out according to the instructions of the kit.
(2) Preparation of cells for transfection

(i) CHO-K1 cells were resuscitated, $6\times10^6$ cells were inoculated into 12 ml CD-CHO medium (containing 6 mM GlutaMAX) at a density of $0.5\times10^6$/ml, and the resultant was subjected to shake cultivation in 5% $CO_2$, at 37°C and 135 rpm.
(ii) On the day before transfection, the cell density was adjusted to $0.5\times10^6$ /ml, and the resultant was subjected to shake cultivation in 5% $CO_2$, at 37°C and 135 rpm.

(3) Electroporation transfection

(i) The cell concentration was measured by cell counting to ensure a cell viability of 95% or more.
(ii) $1\times10^7$ cells were taken, centrifuged at 1,000 rpm for 5 min, the supernatant was discarded, the cells were suspended with fresh CD-CHO medium, the resultant was centrifuged at 1,000 rpm for 5 min, and the supernatant was discarded. Washing was repeated once again.
(iii) Cells were suspended with 0.7 ml CD-CHO medium, 40 μg of expression vector was added to be mix welled and the resultant was transferred to a 0.4 cm electroporation cuvette for electroporation.
(iv) The cells were quickly transferred to CD-CHO medium (without GlutaMAX) after electroporation, and plated in a 96-well plate, and cultured in 5% $CO_2$ at 37°C.
(v) 24 hours after transfection, MSX was added to each well to a final concentration of 50 μM, and the resultant was subjected to cultivation in 5% $CO_2$ at 37°C.
(vi) Monoclonal cell strains that highly express bispecific antibodies were picked out to perform fed-batch fermentation and the supernatant was collected after 14 days of culturing.

4. Purification of bispecific antibodies

(1) Pretreatment of feed

The supernatant of the fermentation culture was centrifuged at 2,000 rpm for 10 min, and then filtered with a 0.22 μM filter membrane.

(2) Affinity chromatography

A Mabselect SuRe affinity chromatography column (purchased from GE, Catalog No. 18-5438-02) was used to capture the antibodies in the pretreated fermentation broth, an equilibration buffer (10 mM PB, 0.1 M NaCl, pH 7.0) was used to fully equilibrate the chromatography column, and the pretreated fermentation broth was allowed to pass through the affinity chromatography column, and elution was performed with an elution buffer (0.1 M citric acid, pH 3.0).

(3) Cation exchange chromatography

The sample prepared by affinity chromatography was further subjected to purification by SP cation exchange chromatography. The cation exchange column was purchased from GE (17-1014-01, 17-1014-03). After equilibration of the chromatography column with an equilibration buffer (50 mM PBS, pH 5.5), the sample was allowed to pass through the SP column for binding, and then linear elution was performed with 20 column volumes of an elution buffer (50 mM PBS, 1.0 M NaCl, pH 5.5).

(4) Anion exchange chromatography

After purification by SP cation exchange chromatography, the resultant was further allowed to pass through an ion exchange Q-Sepharose column (purchased from GE, Catalog Nos: 17-1153-01, 17-1154-01), and the buffer used was 50 mM PBS at pH 5.5.

The purified bispecific antibodies YK001 and YK002 were tested by SDS-PAGE and HPLC-SEC. The result of SDS-PAGE is shown in Figure 3, the test result of reduced SDS-PAGE electrophoresis of YK001 is shown in A of Figure 3, and the test result of non-reduced SDS-PAGE electrophoresis of YK001 is shown in B of Figure 3. The test result of reduced SDS-PAGE electrophoresis of YK002 is shown in C of FIG. 3, and the test result of non-reduced SDS-PAGE electrophoresis of YK002 is shown in D of FIG. 3. The test result of HPLC-SEC is shown in Figure 4, wherein the SEC test result of YK001 is shown in A of Figure 4, and the SEC test result of YK002 is shown in B of Figure 4. The test results show that the bispecific antibodies YK001 and YK002 are successfully prepared after expression and purification, and the purity of the purified bispecific antibodies is 95% or more.

**Example 3:** Determination of the binding activity of bispecific antibodies to tumor cells and immune cells

Raji cells (purchased from ATCC, CCL-86) were used as CD19-positive cells, T cells were used as CD3-positive cells, and the binding activity of the bispecific antibody of the present invention to target antigens of CD19-expressing tumor cells and CD3-expressing immune cells was detected by flow cytometry.

1. Detection of the binding activity of bispecific antibodies to Raji cells by flow cytometry

(1) Collecting Raji cells: cells were collected at $1 \times 10^6$ cells/tube.
(2) Rinsing the cells: the cells were rinsed once with 1 ml staining buffer (PBS containing 0.5% w/v BSA + 2 mM EDTA), the resultant was centrifuged at $350 \times g$ at 4°C for 5 min, and then cells were resuspended with 200 μl staining buffer.
(3) Bs-antibody binding: bispecific antibodies YK001 and YK002 were added to a concentration of 5 μg/ml, respectively, and the resultant was subjected to incubation on ice for 45 min.
(4) Rinsing the cells: 1 ml staining buffer was added to the cell suspension to mix well, and centrifuged at $350 \times g$ at 4°C for 5 min, the supernatant was removed, and the resultant was rinsed once again. After centrifugation, cells were resuspended with 100 μl staining buffer.
(5) 5 μl of Biolegend antibody (PE anti-human IgG Fc Antibody, Biolegend, 409304) was added to a sample tube, isotype control (PE Mouse IgG2a, κ Isotype Ctrl (FC) Antibody, Biolegend, 400213) was added to an isotype control tube, and the resultants were subjected to incubation on ice in dark for 15 min.
(6) Rinsing the cells: 1 ml staining buffer was added to the cell suspension to mix well, the resultant was centrifuged at $350 \times g$ at 4°C for 5 min, the supernatant was removed, and the resultant was rinsed once again.

(7) Detection with a flow cytometer: After resuspending the cells with 200 μl PBS, the resultant was subjected to detection with a flow cytometer.

The results of flow cytometry were shown in Figure 5, wherein the detection results of binding of YK001 to Raji cells are shown in A, B and C of Figure 5, and the detection results of binding of YK002 to Raji cells are shown in D, E and F of Figure 5. The results show that both bispecific antibodies YK001 and YK002 can specifically bind to Raji cells, that is, the bispecific antibody fusion protein retains the binding function of the monoclonal antibody Anti-CD 19.
2. Detection of the binding activity of bispecific antibodies to T cells by means of flow cytometry

(1) Collecting T cells: cells were collected at 1 × $10^6$ cells/tube.
(2) Rinsing the cells: the cells were rinsed once with 1 ml staining buffer (PBS containing 0.5% w/v BSA + 2 mM EDTA), the resultant was centrifuged at 350 × g at 4°C for 5 min, and then the cells were resuspended with 200 μl staining buffer.
(3) Bs-antibody binding: bispecific antibodies YK001 and YK002 were added to a concentration of 5 μg/ml, respectively, and the resultant was subjected to incubation on ice for 45 min.
(4) Rinsing the cells: 1 ml staining buffer was added to the cell suspension to mix well, the resultant was centrifuged at 350 × g at 4°C for 5 min, the supernatant was removed, and the resultant was rinsed once again. After centrifugation, the cells were resuspended with 100 μl staining buffer.
(5) 5 μl of Biolegend antibody (PE anti-human IgG Fc Antibody, Biolegend, 409304) was added to a sample tube, isotype control (PE Mouse IgG2a, κ Isotype Ctrl (FC) Antibody, Biolegend, 400213) was added to an isotype control tube, and the resultants were subjected to incubation on ice in dark for 15 min.
(6) Rinsing the cells: 1 ml staining buffer was added to the cell suspension to mix well, the resultant was centrifuged at 350 × g at 4°C for 5 min, the supernatant was removed, and the resultant was rinsed once again.
(7) Detection with a flow cytometer: After resuspending the cells with 200 μl PBS, the

resultant was subjected to detection with a flow cytometer.

The results of flow cytometry were shown in Figure 6, wherein the detection results of binding of YK001 to T cells are shown in A and B of Figure 6, and the detection results of binding of YK002 to T cells are shown in C and D of Figure 6. The results show that both bispecific antibodies YK001 and YK002 can specifically bind to T cells, that is, the bispecific antibody fusion protein retains the binding function of the single-chain antibody Anti-CD3.

**Example 4: Detection of in-vitro cell killing efficiency mediated by bispecific antibodies**

In the present Example, Raji-Luc cells were used as target cells, PBMCs were used as immune effector cells, and the effect of killing the target cells mediated by bispecific antibodies YK001 and YK002 was detected, with anti-CD3 monoclonal antibody and anti-CD 19 monoclonal antibody and 0527 × CD3 bispecific antibody as control.

1. Preparation of target cells

As target cells, Raji-Luc cells (luciferase-labeled Raji cells) were counted after mixing well by pipetting up and down, centrifuged at 1,000 rpm for 5 min, and washed once with PBS. After centrifugation and washing of the target cells, the density was adjusted to 0.2 × $10^6$/ml with GT-T551 culture medium, 50 μl of the resultant was added to each well with 10,000 cells in each well.

2. Preparation of PBMCs

PBMCs were used as effector cells. PBMCs frozen in a liquid nitrogen tank were taken out (referring to cell cryopreservation and resuscitation), thawed and added to a 15 ml centrifuge tube containing PBS or GT-T551 culture medium, and centrifuged at 1,000 rpm for 5 min. The cells were washed twice with PBS or GT-T551 culture medium and counted, the activity and density of cells were detected, and the density of living cells was adjusted to 2 × $10^6$/ml. 50 μl of the resultant was added to each well with 100,000 cells in each well.

3. Dilution of antibodies

The bispecific antibodies YK001 and YK002 were diluted with GT-T551 culture medium, respectively, and the initial concentration of the antibodies YK001 and YK002 was adjusted to 10 nM. The resultant was diluted sequentially at a ratio of 1:5. 100 μl of the diluted antibody was added to the cells prepared above in a 96-well plate to mix well, the 96-well plate was put back into the incubator, and the killing effect was detected after 18 hours.

4. Detection

Since the Luciferase gene was carried by Raji target cells, the efficiency of killing the target cells was detected by the LUMINEX method.

Steady-GLO (Promega) was used as a substrate. After thawed, the buffer in the kit was added to the substrate powder to mix well, and the resultant was sub-packed with 5 ml or 10 ml for each package to complete reconstruction of the steady-GLO substrate.

After the co-cultured cells were mixed well by pipetting up and down, 100 μl was taken and transferred to an opaque white plate, then 100 μl of the reconstructed steady-GLO substrate was added, the resultant was tapped to mix well, and detected by a plate reader after standing for 5 minutes. The detection instrument was synergy HT.

5. Data processing

The calculation formula for the killing ratio of the target cell is as follows:

$$\text{Killing ratio of target cells} = 100 \times (\text{Only target - test well})/\text{Only target}.$$

The antibody concentration corresponding to the killing ratio of target cells in all detection wells was converted to log10, which was used as the abscissa, and the killing ratio was used as the ordinate to make a graph. The results were shown in Figure 7. The results were analyzed by the software Graphpad Prism 7.0, the IC50 of the bispecific antibody was calculated, and the results were shown in Table 2. The results show that compared with control antibodies (anti-CD3 monoclonal antibody, anti-CD 19 monoclonal antibody and 0527 × CD3 bispecific antibody), both bispecific antibodies YK001 and YK002 can effectively mediate PBMC to kill the tumor cell line Raji-Luc, as a single molecular, both YK001 and YK002 have the biological functions of Anti-CD 19 and Anti-CD3 monoclonal antibodies at the same time, and the efficacy of killing target cells mediated by YK001 is higher than that of YK002.

Table 2: IC50 of target cell killing mediated by bispecific antibodies YK001 and YK002

| | Anti-CD3 | Anti-CD 19 | YK001 | YK002 | 0527 × CD3 |
|---|---|---|---|---|---|
| IC50 (nM) | 0.02866 | N/A | 0.0004193 | 0.002445 | ~0.4051 |

Although the general description and specific embodiments have been used to describe the present invention in detail above, it is obvious to a person skilled in the art that some modifications or improvements can be made based on the present invention. Therefore, these modifications or improvements made without departing from the spirit of the present invention fall within the scope of protection of the present invention.

## Industrial applicability

The present invention provides a bispecific antibody, a preparation method thereof and a use thereof. The bispecific antibody of the present invention comprises a monoclonal antibody unit and a single-chain antibody unit, wherein, the monoclonal antibody unit comprises two complete light chain-heavy chain pairs, and can specifically bind to a surface antigen of a tumor cell; the single-chain antibody unit comprises two single-chain antibodies, and the single-chain antibody comprises a heavy chain variable region and a light chain variable region, and can specifically bind to a surface antigen of an immune cell. The bispecific antibody provided in the present invention is of a symmetric structure formed by linkage in any one of the following modes: (1) C-ends of the two single-chain antibodies are respectively linked to N-ends of two heavy chains of a monoclonal antibody through a linker peptide; (2) N-ends of the two single-chain antibodies are respectively linked to C-ends of the two heavy chains of the monoclonal antibody through a linker peptide. The bispecific antibody of the present invention can simultaneously bind to the immune cell and the tumor cell, mediate a directed immune response, and effectively kill the tumor cell, with good economic value and application prospects.

## Claims

1. A bispecific antibody that binds to CD19 and CD3, wherein the bispecific antibody comprises (a) a monoclonal antibody unit and (b) a single-chain antibody unit; the monoclonal antibody unit consists of two complete light chain-heavy chain pairs, and can specifically bind to CD19; the single-chain antibody unit comprises two single-chain antibodies; the single-chain antibody comprises a heavy chain variable region and a light chain variable region, and can specifically bind to CD3; the bispecific antibody has a symmetric structure formed by linkage in any one of the following modes:

   (1) the C-ends of the two single-chain antibodies are respectively linked to the N-ends of two heavy chains of the monoclonal antibody through a linker peptide; and
   (2) N-ends of the two single-chain antibodies are respectively linked to C-ends of the two heavy chains of the monoclonal antibody through a linker peptide.

2. The bispecific antibody according to claim 1, wherein the amino acid sequence of the linker peptide is (GGGGX)n, wherein X is Gly or Ser, and n is a natural number selected from 1 to 4;
   preferably, the amino acid sequence of the linker peptide is represented by SEQ ID NO. 13.

3. The bispecific antibody according to claim 1 or 2, wherein,

   the light chain sequence of the single-chain antibody is represented by SEQ ID NO. 5 or represented by SEQ ID NO. 9;
   the heavy chain sequence of the single-chain antibody is represented by SEQ ID NO. 6 or represented by SEQ ID NO. 10;
   preferably, the light chain and the heavy chain of the single-chain antibody constitute a fusion peptide, and the sequence of the fusion peptide is any one of the follows:

   (1) when C-ends of the two single-chain antibodies are respectively linked to N-ends of two heavy chains of the monoclonal antibody through a linker peptide, the sequence of the fusion peptide is represented by SEQ ID NO. 16; and
   (2) when N-ends of the two single-chain antibodies are respectively linked to to C-ends of the two heavy chains of the monoclonal antibody through a linker peptide, the sequence of the fusion peptide is represented by SEQ ID NO. 17.

4. The bispecific antibody according to claim 3, wherein the bispecific antibody is a murine antibody, a humanized antibody, a chimeric antibody or a recombinant antibody.

5. The bispecific antibody according to claim 3 or 4, wherein the light chain and the heavy chain of the monoclonal antibody are connected by a disulfide bond;

   Fc fragment of the monoclonal antibody is a Fc fragment of a human or humanized antibody, and the human or humanized antibody is one of IgG1, IgG2, IgG3 or IgG4;
   preferably, the Fc fragment of the monoclonal antibody is a Fc fragment of a human or humanized IgG4 antibody;

more preferably, a full-length sequence of the light chain of the monoclonal antibody is represented by SEQ ID NO. 3; and a full-length sequence of the heavy chain of the monoclonal antibody is represented by SEQ ID NO. 1 or SEQ ID NO. 20.

**6.** A gene encoding the bispecific antibody of any one of claims 1 to 5,

preferably, a gene sequence coding a full-length light chain of the monoclonal antibody is represented by SEQ ID NO. 4; and/or,
a gene sequence coding a full-length heavy chain of the monoclonal antibody is represented by SEQ ID NO. 2 or represented by SEQ ID NO. 21; and/or,
when C-ends of the two single-chain antibodies are respectively linked to N-ends of two heavy chains of the monoclonal antibody through a linker peptide, a gene sequence coding the single-chain antibody is represented by SEQ ID NO. 14; and when N-ends of the two single-chain antibodies are respectively linked to C-ends of the two heavy chains of the monoclonal antibody through a linker peptide, a gene sequence coding the single-chain antibody is represented by SEQ ID NO. 15.

**7.** A biological material comprising the gene of claim 6, wherein the biological material comprises a recombinant DNA, an expression cassette, a vector, a host cell, an engineered bacterium or a cell line.

**8.** A preparation method of the bispecific antibody according to any one of claims 1 to 5, wherein the method comprises: constructing an expression vector containing a coding gene of the single-chain antibody and the monoclonal antibody; introducing the expression vector into a host cell to obtain a host cell stably expressing the bispecific antibody; culturing the host cell, and obtaining the bispecific antibody by separation and purification.

**9.** A pharmaceutical composition, wherein the pharmaceutical composition comprises the bispecific antibody of any one of claims 1 to 5.

**10.** Any one of the following uses of the bispecific antibody according to any one of claims 1 to 5 or the gene according to claim 6 or the biological material according to claim 7:

(1) use in the preparation of a drug for prevention or treatment of a CD 19-expressing B cell-related disease;
(2) use in the preparation of a drug for prevention or treatment of a disease with CD 19 as a target;
(3) use in the preparation of a drug for killing CD19-expressing cells; and
(4) use in the preparation of a detection reagent for CD19 and/or CD3;

preferably, the CD19-expressing B cell-related disease include B cell-related tumors and autoimmune diseases caused by B cells.

Figure 1

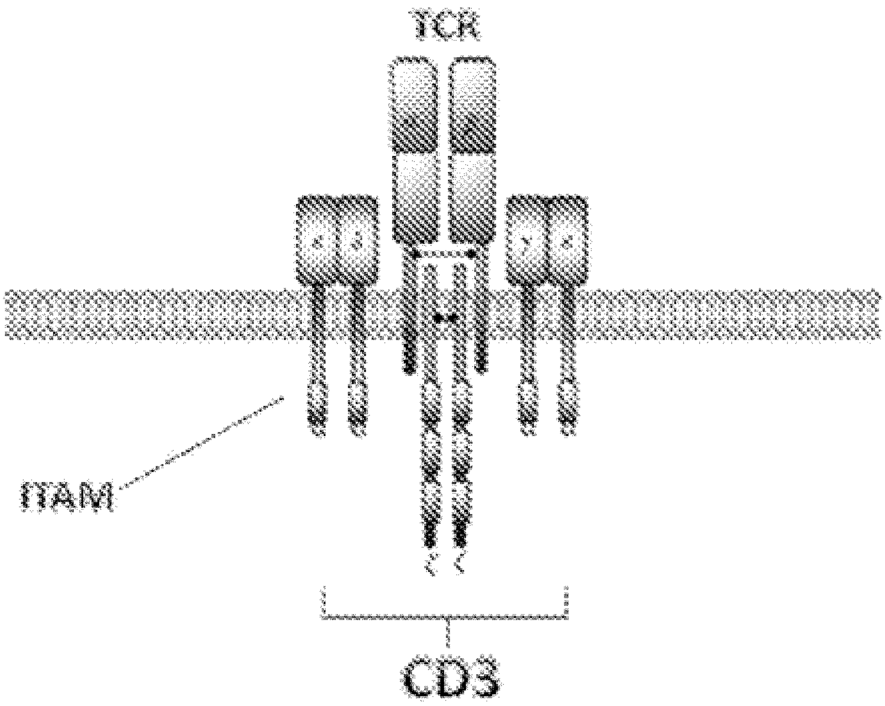


Figure 2

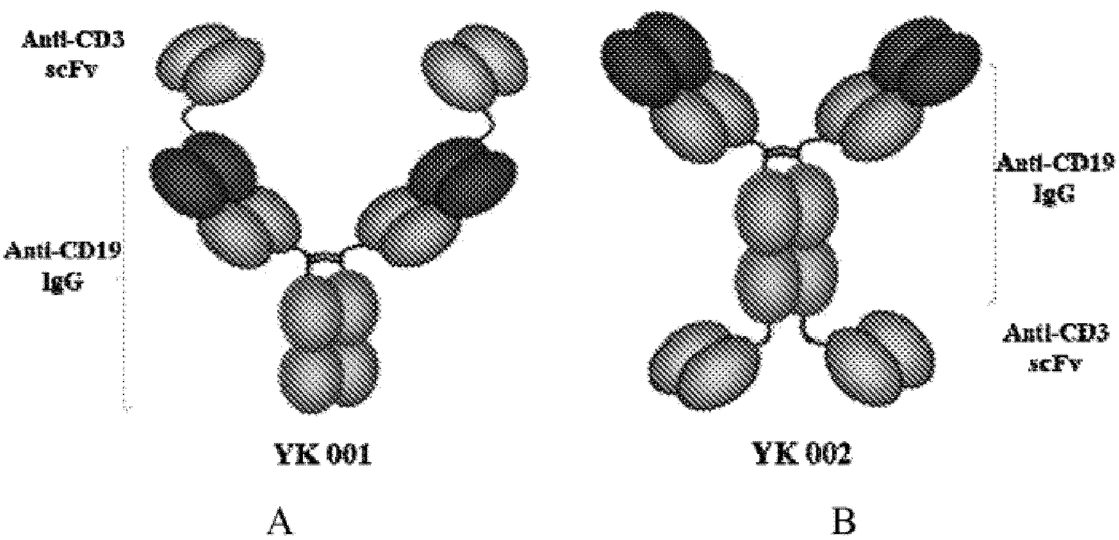

Figure 3

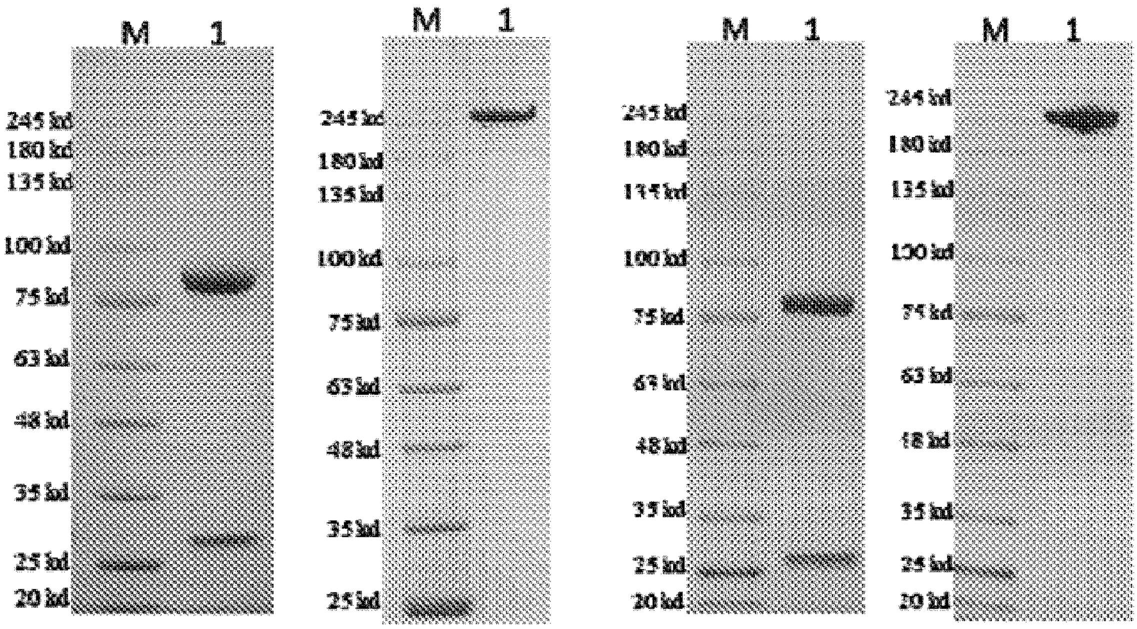


A B C D

Figure 4

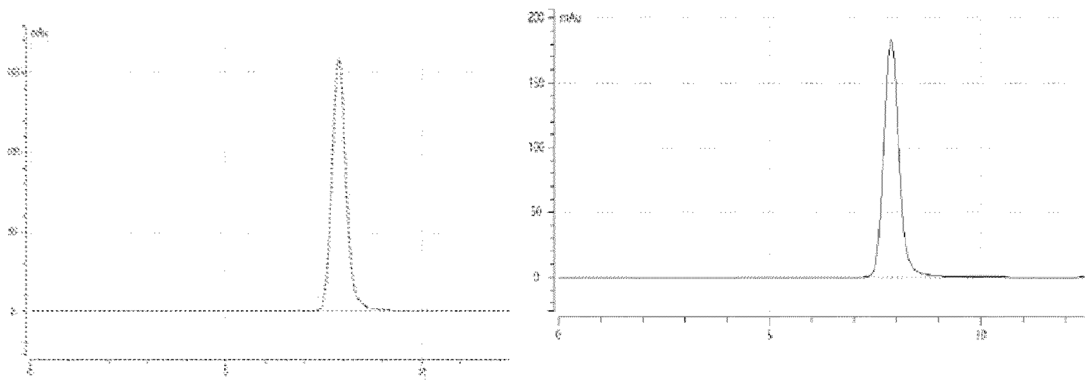

| Peak number | Retention time (min) | Peak area (mAU*S) | area% | Peak height |
|---|---|---|---|---|
| 1 | 7.890 | 4008.80344 | 100 | 158.109 |

| Peak number | Retention time (min) | Peak area (mAU*S) | area% | Peak height |
|---|---|---|---|---|
| 1 | 7.878 | 4556.02845 | 100 | 183.187 |

Figure 5

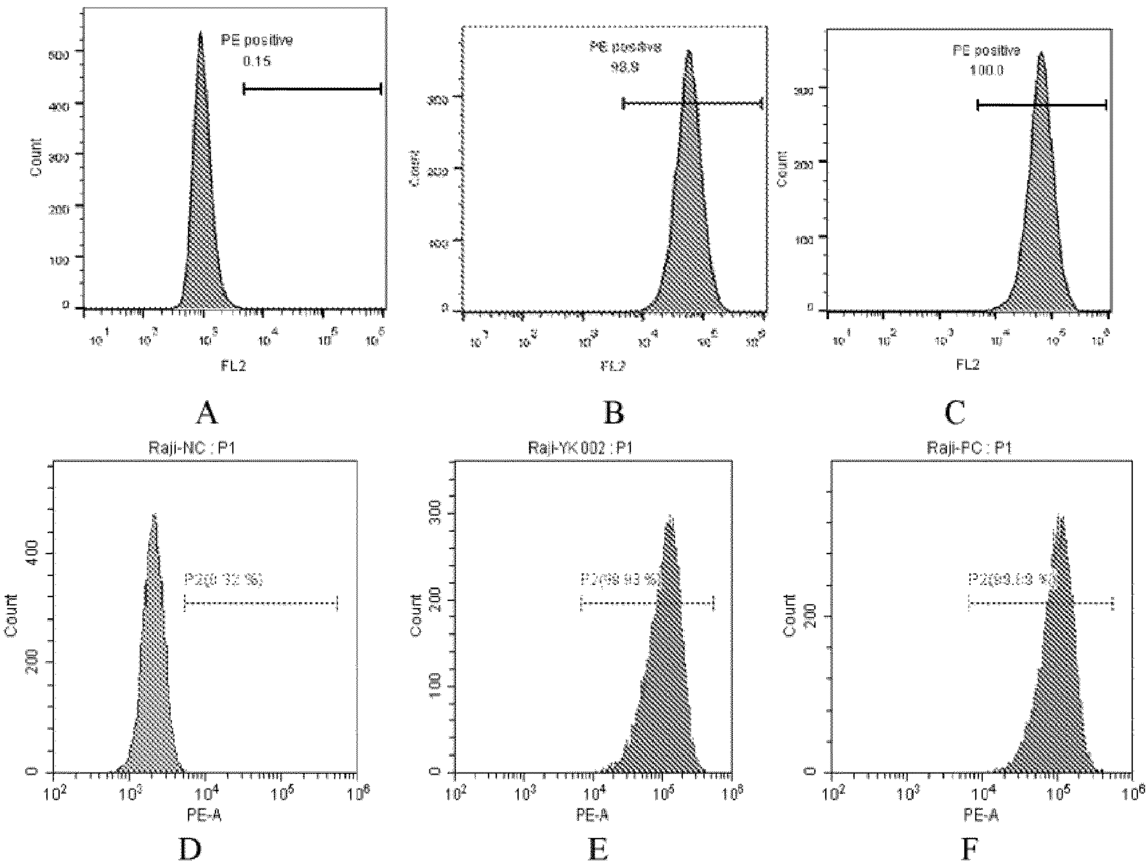
PE positive
0.15
Count
FL2
PE positive
Count
FL2
PE positive
100.0
Count
FL2
A
B
C
Raji-NC : P1
Raji-YK002 : P1
Raji-PC : P1
Count
PE-A
D
E
F

Figure 6

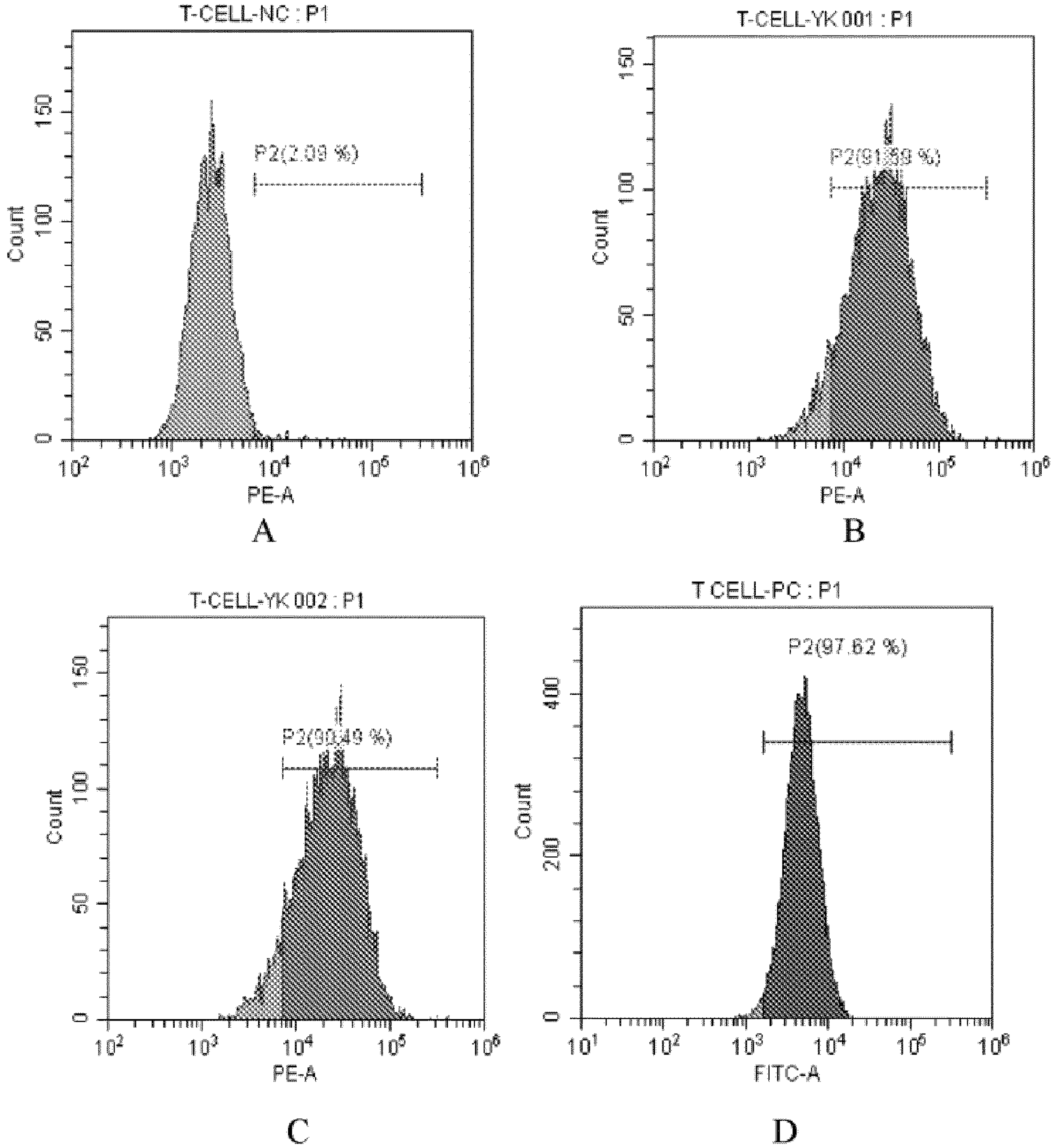
T-CELL-NC : P1
P2(2.09 %)
Count
PE-A
A
T-CELL-YK 001 : P1
Count
PE-A
B
T-CELL-YK 002 : P1
Count
PE-A
C
T CELL-PC : P1
P2(97.62 %)
Count
FITC-A
D

Figure 7

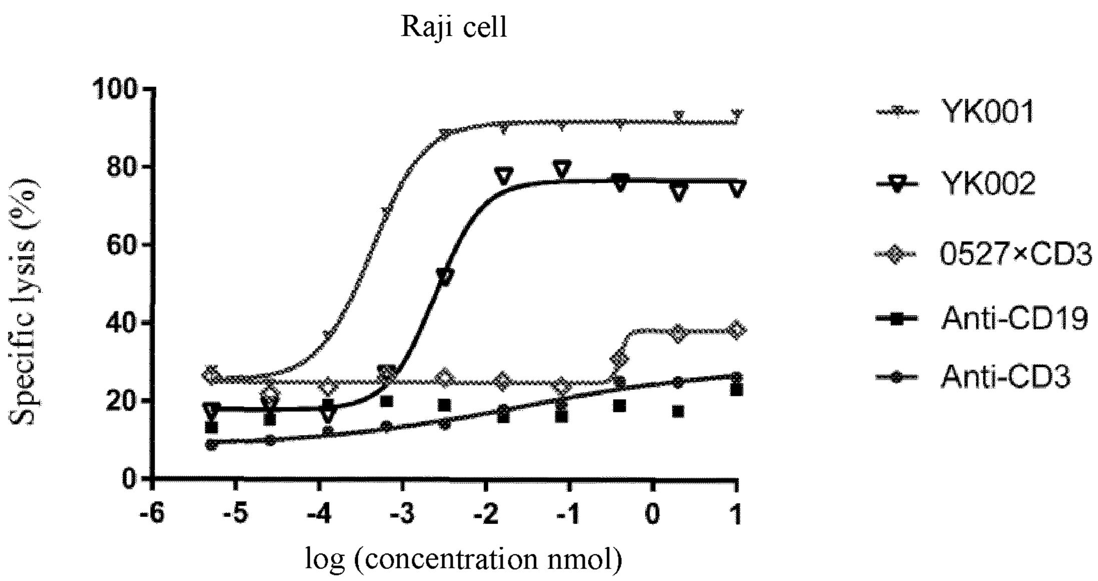
Raji cell
Specific lysis (%)
100
80
60
40
20
0
-6
-5
-4
-3
-2
-1
0
1
log (concentration nmol)
YK001
YK002
0527×CD3
Anti-CD19
Anti-CD3

## INTERNATIONAL SEARCH REPORT

International application No.
**PCT/CN2020/076516**

**A. CLASSIFICATION OF SUBJECT MATTER**

C07K 16/46(2006.01)i; C12N 15/13(2006.01)i; A61K 39/395(2006.01)i; A61P 35/00(2006.01)i; A61P 37/02(2006.01)i; G01N 33/68(2006.01)i

According to International Patent Classification (IPC) or to both national classification and IPC

**B. FIELDS SEARCHED**

Minimum documentation searched (classification system followed by classification symbols)

C07K16; A61K; C12N; A61P; G01N33

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science, GenBank, 中国专利生物序列检索系统, CTCMPD and Searching System thereof: 抗体, 单抗, 双特异性, 单链, antibody, monoclonal, IgG, bispecific, single chain, scFv, CD3, CD19, SEQ ID Nos: 5, 6, 9 and 10 of the present application.

**C. DOCUMENTS CONSIDERED TO BE RELEVANT**

| Category* | Citation of document, with indication, where appropriate, of the relevant passages | Relevant to claim No. |
|---|---|---|
| PX | CN 109776683 A (EXCYTE (BEIJING) PHARMACEUTICAL TECHNOLOGY DEVELOPMENT CO., LTD.) 21 May 2019 (2019-05-21) entire document, particularly the claims | 1-10 |
| X | CN 108690138 A (GMAX BIOPHARM (HANGZHOU) CO., LTD.) 23 October 2018 (2018-10-23) entire document, particularly claims, description, paragraphs [0008]-[0013] and [0091]-[0104], sequence list, and the abstract | 1, 2, 6-10 |
| Y | CN 108690138 A (GMAX BIOPHARM (HANGZHOU) CO., LTD.) 23 October 2018 (2018-10-23) entire document, particularly claims, description, paragraphs [0008]-[0013] and [0091]-[0104], sequence list, and the abstract | 3-10 |
| Y | CN 107406509 A (BIOTECNOL LTD.) 28 November 2017 (2017-11-28) entire document, particularly claims, description, paragraphs [0153] and [0154], sequence list, and the abstract | 3-10 |
| Y | CN 106062206 A (ZYMEWORKS INC.) 26 October 2016 (2016-10-26) entire document, particularly claims, sequence list, and the abstract | 3-10 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 April 2020** | **29 May 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT

International application No.
PCT/CN2020/076516

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2020/076516**

| Patent document cited in search report | Publication date (day/month/year) | Patent family member(s) | Publication date (day/month/year) |
|---|---|---|---|
| CN 109776683 A | 21 May 2019 | None | |
| CN 108690138 A | 23 October 2018 | WO 2018188612 A1 | 18 October 2018 |
| CN 107406509 A | 28 November 2017 | AU 2015366166 A1 | 29 June 2017 |
| | | JP 2018500025 A | 11 January 2018 |
| | | KR 20170094319 A | 17 August 2017 |
| | | GB 201709537 D0 | 02 August 2017 |
| | | CA 2970986 A1 | 23 June 2016 |
| | | EP 3233917 A1 | 25 October 2017 |
| | | WO 2016097408 A1 | 23 June 2016 |
| | | GB 2549632 A | 25 October 2017 |
| | | BR 112017012910 A2 | 06 February 2018 |
| | | US 2017342160 A1 | 30 November 2017 |
| | | MX 2017007826 A | 13 February 2018 |
| CN 106062206 A | 26 October 2016 | KR 20160107304 A | 13 September 2016 |
| | | CA 2936785 A1 | 23 July 2015 |
| | | WO 2015109131 A2 | 23 July 2015 |
| | | WO 2015109131 A3 | 12 November 2015 |
| | | RU 2016132863 A | 20 February 2018 |
| | | BR 112016016114 A2 | 22 May 2018 |
| | | MX 2016009050 A | 09 December 2016 |
| | | JP 2017504328 A | 09 February 2017 |
| | | AU 2015206407 A1 | 18 August 2016 |
| | | EP 3094737 A4 | 09 August 2017 |
| | | US 2016326249 A1 | 10 November 2016 |
| | | EP 3094737 A2 | 23 November 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201910209330